# EUROPEAN PATENT SPECIFICATION

(11) **EP 1 114 185 B1**
(45) Date of publication and mention of the grant of the patent: **22.04.2009**
(21) Application number: 99947604.7
(22) Date of filing: 15.09.1999
(51) Int. Cl.: C12Q 1/68

(54) **METHOD OF ISOLATING PRIMER EXTENSION PRODUCTS WITH MODULAR OLIGONUCLEOTIDES**
METHODE ZUR ISOLIERUNG VON "PRIMER EXTENSION" - PRODUKTEN MIT MODULAREN OLIGONUCLEOTIDEN
PROCEDE D'ISOLATION DE PRODUITS D'EXTENSION D'AMORCES AU MOYEN D'OLIGONUCLEOTIDES MODULAIRES

(30) Priority: 15.09.1998 US 153242; 16.09.1998 GB 9820185
(43) Date of publication of application: 11.07.2001
(73) Proprietor: Invitrogen Dynal AS, 0309 Oslo (NO)
(72) Inventor: LUNDEBERG, Joakim CEMU Bioteknik AB, 100 44 Stockholm (SE); UHLEN, Mathias CEMU Bioteknik AB, 100 44 Stockholm (SE)
(74) Representative: Couchman, Jonathan Hugh
(86) International application number: PCT/GB1999/003056
(87) International publication number: WO 2000/015842

(56) References cited:
- WO-A-93/20232
- WO-A-98/13522
- WO-A-98/14610
- US-A- 5 149 625
- KOTLER L E ET AL: "DNA SEQUENCING: MODULATOR PRIMERS ASSEMBLED FROM A LIBRARY OF HEXAMERS OR PENTAMERS" PROCEEDINGS OF THE NATIONAL ACADEMY OF SCIENCES OF USA,US,NATIONAL ACADEMY OF SCIENCE. WASHINGTON, vol. 90, 1 May 1993 (1993-05-01), pages 4241-4245, XP002054939 ISSN: 0027-8424 cited in the application

## Description

The present invention relates to a method of improving the binding of a series of consecutive nucleotide bases to a complementary nucleic acid molecule, especially for use in improving the binding of capture oligonucleotides, in methods for isolating primer extension products, such as sequencing products, modular oligonucleotides and kits for performing methods of the invention.

The binding of complementary nucleotide bases to one another represents one of the most significant and fundamental findings in science this century and heralded the rapid development of the field of biochemistry. Whilst allowing an understanding of the mechanisms underlying the continuation of life, the discovery has also provided the basis for the development of valuable molecular biological tools.

The isolation and sequencing of naturally occurring nucleic acid molecules is a common goal for molecular biologists. The use of complementary oligonucleotides to isolate nucleic acid molecules is commonplace.
Similarly, complementary oligonucleotides are frequently used to bind single-stranded nucleic acid molecules and act as primers for extension reactions to produce complementary strands to the template and forms the basis of such experimental procedures as polymerase chain reaction (PCR) and sequencing reactions.

However, the specificity of binding of oligonucleotides to template or target DNA depends on a number of parameters any one of which may result in poor efficiency of binding and consequently poor experimental results. The specificity of the interaction may conveniently be determined by the assessment of Tₘ, the temperature at which duplexes dissociate. This is however also dependent on other parameters, for example the buffer in which the reaction is performed. For a particular experimental system, Tₘ will be affected by various factors including the extent of complementarity, the sequence of the target and/or oligonucleotide, derivatization of the oligonucleotide and length of the oligonucleotide. The binding of oligonucleotides may therefore be improved, as evidenced by an increased Tₘ under the same experimental conditions, by altering these parameters. However, the variation which may be achieved by altering these parameters is limited.

It has since been found that modular probes or primers composed of at least two modules (oligonucleotides) which bind to adjacent regions of target DNA exhibit improved binding relative to a single oligonucleotide spanning the same length as the separate modules (see WO98/13522). For example, it has been found that two adjacent 18-mer oligonucleotides bind more efficiently to target DNA than the composite 36-mer oligonucleotide.

The use of primers composed of adjacent modules for sequencing purposes has been described previously (Kotler et al., 1993, Proc. Natl. Acad. Sci. USA, 90, p4241-4245; Kieleczawa et al., 1992, Science, 258, p1787-1791 and Szybalski, 1990, Gene, 90, p177-178). However, in these cases the modular primers were used to replace longer primers such that libraries of all sequences of the shorter primers could realistically be pre-synthesized as they had fewer possible sequence permutations than longer primers. In all cases, the modular primers were only shown to have, in sequencing reactions under the same conditions, efficacy as good as the longer primers. In contrast, in the invention described in WO98/13522, even better binding is achieved, when a single oligonucleotide is split into separate components. Furthermore, work prior to WO98/13522 indicates that the effect of modular primers may only be achieved if the modules do not have a single (or more) base(s) between them when bound to the template. For improved binding as described in WO98/13522 and herein, no such restriction is applicable although even better binding is observed when no gaps exist between the modules.

Lin et al. (Lin et al., 1989, Biochemistry, 28, p1054-1061) describes that a cooperative effect, probably due to base-stacking, occurs between adjacently bound oligonucleotides which increases the Tₘ. This however is compared only to the binding of one of the modules of the modular primer and not a composite primer of the full length of the modular primer. The findings presented in WO98/13522 thus presented a considerable advance over the prior art and had many applications for instances in which improved binding of an oligonucleotide to a target nucleic acid molecule was required.

The method of WO98/13522 which is also described herein may be used for any application in which improved binding of nucleotide bases, preferably in the form of an oligonucleotide, to a target nucleic acid molecule is required. Whilst not wishing to be bound by theory, it appears that the use of modular oligonucleotides allows the disruption of tertiary structures of nucleic acid molecules which are present not only in tRNA but also in other nucleic acid molecules. Such tertiary structures do not appear to be as effectively disrupted using longer oligonucleotides in which the parts of the modular oligonucleotide are synthesized together as a single molecule. Thus, applications which require improved binding to areas of nucleic acid molecules with tertiary structure which would prevent or impair binding of an oligonucleotide to this region, will benefit from this invention. The invention in WO98/13522 therefore describes applications in which the modular oligonucleotide serves as a primer in methods which involve replication, amplification, transcription, reverse transcription and/or sequencing, or in which the modular oligonucleotide serves as a probe for detection and/or capture or isolation of target nucleic acid molecules. It will be appreciated that in appropriate circumstances modular oligonucleotides may serve both of the aforementioned functions, e.g. by serving both as a primer and also as a capture/detection probe for the nucleic acid products e.g. amplified DNA, thus produced.

It has now been found that the modular oligonucleotides described in WO98/13522 offer unique advantages in the isolation of primer extension products, such as sequencing products generated by primers. Such products need to be purified and/or enriched after synthesis before loading onto an electrophoresis system. In large genomic sequencing projects, where large numbers of samples are handled, automation is an important requirement. Assays that include precipitation, extraction or centrifugation steps are difficult to automate. Although alcohol precipitation is routinely used in laboratory protocols, this is not easy to automate. The use of modular probes allows the capture of such products as generated by, for example, traditional T7 DNA polymerase sequencing or cycle sequencing protocols. Thus these modular oligonucleotides allow the development of a method of isolating primer extension products, wherein said method comprises at least the step or steps of binding a complementary modular oligonucleotide of at least two parts (modules) to adjacent stretches on said primer extension products, wherein at least one module (capture module) is immobilized or has means for immobilization.

To develop methods suitable for large scale use, it is also important to consider the costs of sequence product purification per sample. Whereas previous methods relied on the purification and concentration of Sanger fragments prior to separation by gel electrophoresis (although spin-columns have also been used), since the use of modular oligonucleotides for isolation relies on a hybridisation event, re-use of the solid support, for example paramagnetic beads is possible.

It will be appreciated that the method described in WO98/13522 may be adapted for use with respect to different primers used to synthesize extension products. Thus, for example, in the primer extension products produced by a particular primer, a region will exist which corresponds to that primer and that region may be used to isolate the primer extension products by producing a modular oligonucleotide complementary to that region.

WO98/14610 describes methods for capturing sequencing ladders or PCR amplification products by using non-modular primers that allow recovery of the polymerase reaction products by binding of a recovery molecule to the primer region or a region adjacent to the primer in the newly synthesized molecules.

However, such methods have the limitation that free primer and misprimed products will both bind to, and hence be captured by, the modular oligonucleotides. This thus offers little advantage over the use of ethanol precipitation (which would precipitate all such products) other than the potential for automation.

It has however now been found that modular oligonucleotides may be directed to regions of the primer extension products other than the primer-derived regions and such methods offer surprisingly advantageous results.

Thus viewed from a first aspect the present invention provides a method of isolating primer extension products, produced from a template vector, said products containing sequences corresponding or complementary to i) a primer binding region, ii) an insert and iii) vector-derived sequence(s), wherein said method comprises at least the step or steps of binding a modular oligonucleotide of at least two parts (modules) to adjacent stretches on said primer extension products, wherein said modular oligonucleotide is complementary to and capable of binding to said vector-derived sequences of said primer extension products, wherein at least one module (capture module) which is immobilized or has means for immobilization, and further wherein said vector-derived sequence(s) of said primer extension products is/are derived from any region of the vector used to produce the template vector and contains a portion which remains intact after cloning of the insert, regardless of restriction site of a multiple cloning site of said vector into which the insert is cloned, and to which the modular oligonucleotide anneals, and wherein binding of the modular oligonucleotide and the primer binding site and/or insert is not sufficient to allow capture of primers and/ or misprimed products, and further wherein the method results in captured primer extension products.

Preferably, said modular oligonucleotide exhibits improved binding relative to a single oligonucleotide complementary to the region of the target molecule (herein referred to as the primer extension products or sequencing products) spanned by the modular oligonucleotide.

As used herein, the term "improving" with respect to binding is intended to indicate increased specificity, stability or ability to bind to target nucleic acid molecules. "Binding" may be determined according to any method known in the art (for example as described herein) and will, as will be clear to the skilled addressee, be dependent on establishing appropriate buffer, temperature and other conditions. Binding of the modular oligonucleotide may be performed by binding all the parts thereof simultaneously or alternatively, sequential steps involving binding one or more of the modules at each step may be performed. "Complementary" as used herein is intended to encompass any series of consecutive nucleotide bases, oligonucleotide or target/template nucleic acid, as appropriate, which is complementary to the nucleotide sequence of the nucleic acid molecule in question, or its corresponding RNA, DNA, or nucleic acid analog, peptide nucleic acid (PNA). Modules of the modular oligonucleotide may be formed as a composite of the different nucleic acid molecules, e.g. DNA and PNA. Alternatively, individual modules may be composed exclusively of RNA, DNA or PNA, but different modules within the modular probe may be of a different nucleic acid. Thus, for example, a PNA module may be used as a capture probe whereas adjacent modules may be composed of DNA such that extension procedures (e.g. RT-PCR, DNA sequencing etc.) may be performed using the DNA module. Complementarity of the nucleic acid molecules includes within its scope non-absolute complementarity in which some mismatching may occur, although the "complementary" nucleic acids, or oligonucleotides or series of nucleotides, as appropriate, bind to one another under conditions of high stringency. Such oligonucleotides are those which bind under non-stringent conditions (e.g. 6xSSC/50% formamide at room temperature) and washed under conditions of high stringency (e.g. 2xSSC, 65°C), wherein SSC=0.15M NaCl, 0.015M sodium citrate, pH 7.2.

"Nucleic acid molecule" is intended to cover inter alia RNA, mRNA, DNA, cDNA e.g. from retroviral RNA, genomic DNA, mitochondrial DNA etc. and PNA. The DNA may be single or double stranded. When double stranded DNA is used, appropriate procedures may be necessary to allow binding of the modular oligonucleotide, for example by heating to disrupt the structure to the single stranded form. "Target" nucleic acid includes molecules which are isolated according to methods of the invention, ie. primer extension or sequencing products. Nucleotide bases or oligonucleotides which bind to the target nucleic acid molecule may be modified or derivatized, providing they retain the ability to fulfill the complementarity requirements described above. For example, methylated, ethylated or carboxylated bases or other such modified or unusual bases may be used. Alternatively, the nucleic acid backbone may be modified, e.g. PNA units. Alternatively the base may carry a label, for example a hapten such as biotin or a dye. "Oligonucleotides" encompass any piece of DNA (or RNA after reverse transcription), RNA or PNA and extends also to the use of chimers of RNA, DNA and/or PNA.

"Modular oligonucleotide" refers to the primer/probe oligonucleotide which is composed of more than one part. Each part is an oligonucleotide which is referred to as a module of the whole. "Adjacent" as used herein is intended to signify non-overlapping regions of the nucleic acid molecule which lie close to one another, for example are less than 100 or 50 nucleotide bases apart, preferably 10 bases apart, especially preferably less than 2 bases apart, and most preferably without any bases in between, i.e. directly adjacent. Thus, the "single oligonucleotide" referred to above which comprises the modular oligonucleotide may include more nucleotides than the sum of the nucleotide bases in all parts of the modular oligonucleotide as the bases complementary to the region between the binding site of each module of the modular oligonucleotide will also be included in instances in which the modules, when bound, are not directly adjacent.

"Isolating" as used herein is intended to encompass the capture of target nucleic acid, even if this is not removed from the sample in which it is present, ie. physical separation or purification is not necessarily performed. As used herein, "capture" of the target from the sample in which it is contained by binding an oligonucleotide to it, effectively isolates it from other DNA molecules present in the sample. Advantageously however, physical separation or purification is performed. This overcomes problems experienced in the prior art, e.g. in high throughput DNA sequencing instruments such as MegaBACE (Amersham Pharmacia Biotech) and ABI3700 (Applied Biosystems Inc.) in which samples containing the sequencing products are loaded onto capillaries. These capillaries however become clogged if too much template DNA (e.g. plasmid, PCR products, M13) is present in the sample. In preferred aspects of the present invention, in contrast, the sequencing products may be separated from the rest of the sample, e.g. by the use of magnetic beads to which the capture module is, or may be, bound. Thus this method is tolerant to variations in the amount of template present in the sample (see Example 4). The results show that using routine methods of ethanol precipitation, the capillaries are unable to cope with increased amounts of template. In contrast, the isolation method described herein produces good results regardless of the level of template present in the sample.

As referred to herein, reference to a primer binding region, the insert and vector-derived sequences includes sequences which are complementary thereto and it will be appreciated for example that modular oligonucleotides of the invention bind to vector-derived regions of the primer extension product and thus are complementary thereto and have a corresponding sequence to the original vector.

As used herein reference to a "template vector" refers to any piece of nucleic acid from which primer extension products may be produced, ie. susceptible to nucleic acid extension reactions, comprising a primer binding region, an insert (a portion of DNA, e.g. for sequencing) and further regions to which the modular oligonucleotide may be directed. In preferred embodiments said template vectors are vectors which are known in the art, such as pBluescript SK or II SK, pGEM-3Z, 4Z or 5Z, pUC18 or pUC19, RIT27 or RIT28 which comprise additional sequences of varied functionality. In such cases, the "insert" is a portion of nucleic acid which in inserted into a restriction site of the vector through the use of appropriate restriction enzymes. The insert may however be provided in the template vector by other means such as by ligation.

Primer extension products may be prepared directly using a primer site present, or introduced into, the vector or may for example be produced indirectly from the template vector by extension of intermediate products, e.g. extension of products of PCR amplification of the template vector using PCR primers which introduce a further primer site. In such cases the primer site of relevance is the site on the PCR products, ie. the primer site for the final primer extension reactions.

Vector-derived sequences are derived from any portion of the template vector, but excluding (subject to the comments below) the primer binding region and the insert. Reference to "vector" rather than "template vector" thus refers to the vectors without the insert, e.g. any one of the vectors mentioned above.

As mentioned previously, in the present invention modular oligonucleotides are used which avoid isolation of primers or misprimed products. For this purpose, as stated above, such modular oligonucleotides are complementary to and capable of binding to vector-derived sequences. It will however be appreciated that some complementarity may exist between one or more modules of the modular oligonucleotide and the primer binding region and/or the insert providing that as a whole, the complementarity and hence binding is not sufficient to allow capture of primers or misprimed products. Thus for example a portion of one of the modules of the modular oligonucleotide may be complementary to the primer binding sequence (or the insert), e.g. less than 4 base pairs, but this would be insufficient to allow capture of the primer. Furthermore, since the complementarity of the capture module ultimately dictates which molecules are captured, the other non-capture modules of the modular oligonucleotide may be fully complementary to the primer binding sequence (or the insert), but providing the capture module is not sufficiently complementary to the primer binding sequence to allow its capture, undesired products will not be captured. In the latter case it might be necessary to increase the concentration of the modulating modules to account for the binding to unincorporated primers.

In the above cases some complementarity to the primer sequence may occur without the capture of unincorporated primers or misprimed products. Complementarity to the insert can also be tolerated, although in such cases it is desirable to capture primer extension products containing regions corresponding to the insert. Thus to allow their capture, appropriate regions of the modular oligonucleotide may be synthesized as a set of degenerate nucleotides such that all possible variations are covered and thus regardless of the sequence of the insert the necessary binding to the modules will occur. It is however preferred that the modular oligonucleotide is complementary to only vector-derived sequences.

In the design of these modular oligonucleotides, consideration was taken of the two main DNA sequencing chemistries, namely the use of dye terminators, or dye primers, for both cycle sequencing (Taq DNA polymerase or derivative) as well as isothermal protocols (T7 DNA polymerase or derivative), so that the modular oligonucleotides are compatible with either system. Importantly, the immobilised capture probe is not sufficiently complementary to the sequencing primer, which is employed to generate the sequencing products, to allow any sequence corresponding to the primer sequence to be captured. In the case of dye primer chemistry this results in unused labelled dye primer not being co-purified with extended Sanger fragments, which serves to reduce the background in the chromatograms. Furthermore, surprisingly, this also serves to improve the accuracy of sequencing as described later in more detail and in the Examples herein. In the case of dye terminator chemistry (as well as for dye primers) misprimed sequencing products are not purified due to the lack of complementarity between the misprimed product and the capture probe. Again this reduces background and may also improve accuracy of sequencing.

In these new methods, two different approaches have been taken to the design of modular oligonucleotides. Firstly, dedicated or specific modular oligonucleotides have been designed which are specifically appropriate for use in large scale projects in which the same vector and cloning site of the insert are used extensively. In this case the modular oligonucleotides anneal in parts of the multiple cloning site of the vector which are kept intact after cloning of the insert.

Thus, in a preferred feature the present invention provides a method of isolating primer extension products produced from a template vector, said products containing sequences corresponding or complementary to i) a primer binding region, ii) an insert and iii) vector-derived sequence(s) wherein said vector-derived sequence(s) of said primer extension products is derived from any region of the vector used to produce the template vector and contains a portion which remains intact after cloning of the insert into a particular restriction site of the multiple cloning site of said vector and to which the modular oligonucleotide anneals.

For example, for capture of the products of universal primer extension reactions in the forward direction for inserts in the vector pUC18, suitable modular oligonucleotides for use in methods of the invention include modular oligonucleotides having one of the following sequences:
JL-H1/USP + JL-C2/USP (9+9)
   3'-GACGTCCAG-5' + 3'-CTGAGATCT-5'
JL-H2/USP + JL-C1/USP (13+18)
   3'-GTTCGAACGTACG-5' + 3'-GACGTCCAGCTGAGATCT-5'
JL-H2/USP + JL-H1/USP + JL-C2/USP (13+9+9)
   3'-GTTCGAACGTACG-5' + 3'-GACGTCCAG-5' + 3'-CTGAGATCT-5'

Preferably in the above modular oligonucleotides, the last listed module is the capture module (ie. JL-C1/USP, JL-C2/USP) and may bear a moiety for immobilization, preferably a biotin molecule at the 5' end. Thus in a yet still further aspect, the present invention provides methods of the invention for isolating primer extension products, especially of the primer, USP, in the forward direction for inserts in the vector pUC18, wherein said oligonucleotides comprise one of the following nucleotide sequences:
3'-GACGTCCAG-5' + 3'-CTGAGATCT-5'; or
3'-GTTCGAACGTACG-5' + 3'-GACGTCCAGCTGAGATCT-5'; or
3'-GTTCGAACGTACG-5' + 3'-GACGTCCAG-5' + 3'-CTGAGATCT-5',
or analogs or derivatives thereof.

A further example of a dedicated modular oligonucleotide of the invention is provided by oligonucleotides which have the sequence:
5'-ACCCAATTCGCCCTATAG-3' + 5'-TGAGTCGTATTAC-3', or analogs or derivatives thereof. Especially preferably the first stated oligonucleotide behaves as the capture probe and the second oligonucleotide as the modulating module.

Analogs and derivatives as referred to herein include modified or derivatized nucleotide bases or oligonucleotides as referred to previously, which retain their ability to fulfill the complementarity requirements described herein and which include for example, nucleotides bearing labels or means for immobilization. Alternatively, particular bases defined above may be replaced by other non-complementary bases or derivatized bases which do not prevent binding to the target nucleic acid to such an extent as to fall outside the definition of complementarity as described herein.

These modular oligonucleotides and others suitable for use in methods of the invention form further aspects of the invention.

The second approach to the design of suitable modular oligonucleotides to capture primer extension products is the production of generic modular oligonucleotides which are able to bind to and capture primer extension products generated from any insert cloned into the multiple cloning site of a given vector, ie. even the most extreme positioned restriction sites may be used for cloning. Thus in a further preferred feature the present invention provides
a method of isolating primer extension products produced from a template vector, said products containing sequences corresponding or complementary to i) a primer binding region, ii) an insert and iii) vector-derived sequence(s), wherein said method comprises at least the step or steps of binding a modular oligonucleotide of at least two parts (modules) to adjacent stretches on said primer extension products, wherein said modular oligonucleotide is complementary to and capable of binding to said vector-derived sequence, and said vector-derived sequence(s) of said primer extension products is
derived from any region of the vector used to produce the template vector, and
contains a portion which remains intact after cloning of the insert, regardless of the restriction site of the multiple cloning site of said vector into which the insert is cloned, and to which the modular oligonucleotide anneals,
wherein at least one module (capture module) is immobilized or has means for immobilization, preferably wherein said binding is performed at between 40 and 60°C for between 15 to 90 minutes.

The following table provides details of a number of modular oligonucleotides of the generic (and specific) type of the invention which may be used for the collection of primer extension products produced from the specified vectors into which nucleic acid inserts have been cloned.

**Table 1: Generic and specific modular oligos for primer extension products isolation**

| **VECTOR** | **DIRECTION** | **CAPTURE PROBE*** | **MODULATING MODULE** |
|---|---|---|---|
| *GENERIC MODULAR OLIGONUCLEOTIDES* | | | |
| pUC18/pRIT28 | Forward | 5'-NNAAGCTTACT-3' | 5'-GGCCGTCGTTTTACAACG-3' |
| pUC18/pRIT28 | Forward | 5'-NNAAGCTTGGCACT-3' | 5'-GGCCGTCGTTTTACAACG-3' |
| pBluescript, pBluescript II, pGEM3Z and 5Z | Forward | 5'-NNNNAATTCGCCCTATAG-3' | 5'-TGAGTCGTATTAC-3' |
| pUC18 | Reverse | 5'-GAATTCACGGAAATCATG-3' | 5'-GTCATAGCTGTTTCCTGT-3' |
| pUC18 | Reverse | 5'-NNGAATTCGTAATCATGGTC-3' | 5'-ATAGCTGTTTCCTGTGT-3' |
| pBluescript and pBluescript II | Reverse | 5'-TCCAGCTTTTGTTCC-3' | 5'-CTTTAGTGAGGGTTAATT-3' |
| pBluescript and pBluescript II | Reverse | 5'-AGCTCCAGCTTTTGTTCC-3' | 5'-CTTTAGTGAGGGTTAATT-3' |
| pGEM3Z and 5Z | Reverse | 5'-NNTTGAGTATTCTATAG-3' | 5'-TGTCACCTAAATAGCT-3' |

| *SPECIFIC MODULAR OLIGONUCLEOTIDES* | | | |
|---|---|---|---|
| pUC18 | Forward | 5'-TCTAGAGTCGACCTGCAG-3' | 5'-GCATGCAAGCTT-3' |
| pBluescript and pBluescript II | Forward | 5'-ACCCAATTCGCCCTATAG-3' | 5'-TGAGTCGTATTAC-3' |
| pUC18 | Forward | 5'-CGAGCTCGAATTCGTAATC-3' | 5'-ATGGTCATAGCTGTTTCC-3' |

| | | | |
|---|---|---|---|
| * N denotes the production of a family of degenerate oligonucleotides such that all possible permutations are produced | | | |

The means for immobilization may be inherently part of the nucleic acid sequence of the capture module, for example a poly T tail may be provided to bind to a solid support carrying a complementary oligo dA sequence. It will be appreciated that it is inadvisable to use a capture module with a poly A tail to be bound to a support carrying an oligo dT sequence as to do so may lead to the capture of mRNA which may be present in the sample. Other specific sequences which are complementary to sequences which can be attached directly or indirectly to an immobilizing support may also form part of the capture module for the purposes of immobilization.

The above method involves the addition of further nucleotides to the capture module over those required for binding to the target nucleic acid. Extensions in this way are not always necessary and the means for immobilization may be introduced during or post oligonucleotide synthesis to nucleotides of the capture module to allow direct or indirect attachment to an immobilizing support through a binding partner. Conveniently, derivatized nucleotides may be used during synthesis to provide the appropriate first partner of the binding pair. The second partner of the binding pair is then carried on the support. Suitably derivatized capture oligonucleotides thus include those carrying biotin for binding to avidin or streptavidin, carrying epitopes or haptens (eg. digoxigenin) for binding to antibodies (which may be mono- or polyclonal) or antibody fragments or carrying DNA sequences for binding to DNA or PNA binding proteins (eg. the lac I repressor protein binding to a lac operator sequence attached to the oligonucleotide). Other suitable pairings include protein A-antibody, protein G-human serum albumin (HSA) and functional parts thereof. It will be appreciated that either of the partners of the binding pairs noted above, or functional parts thereof, may bind to the oligonucleotide. The streptavidin/biotin binding system is very commonly used in molecular biology, due to the relative ease with which biotin can be incorporated within nucleotide sequences, and indeed the commercial availability of biotin-labelled nucleotides, and thus this represents one preferred method for attachment of the capture module to the support.

Numerous suitable supports for immobilization of oligonucleotides, and methods of attaching nucleotides to them, are well known in the art and widely described in the literature. Thus for example, supports in the form of sheets, gels, filters, membranes, microfibre strips, plates, microtitre wells, tubes, dipsticks, particles, fibres or capillaries may be used, made of a polymeric material for example of agarose, cellulose, alginate, teflon, latex or polystyrene. Particulate materials, especially beads, are generally preferred. For example, sepharose or polystyrene beads may be used. Advantageously, the support may comprise magnetic particles, eg. the superparamagnetic beads produced by Dynal AS (Oslo, Norway) and sold under the trademark DYNABEADS. Chips may be used as solid supports to provide miniature experimental systems as described for example in Nilsson et al. (1995, Anal. Biochem., 224, p400-408).

The solid support may carry functional groups such as hydroxyl, carboxyl, aldehyde or amino groups for the attachment of the capture module. These may in general be provided by treating the support to provide a surface coating of a polymer carrying one of such functional groups, eg. polyurethane together with a polyglycol to provide hydroxyl groups, or a cellulose derivative to provide hydroxyl groups, a polymer of copolymer of acrylic acid or methacrylic acid to provide carboxyl groups or an amino alkylated polymer to provide amino groups. US patent No. 4,654,267 describes the introduction of many such surface coatings.

Alternatively, the support may carry other moieties for attachment, such as avidin or streptavidin, DNA binding proteins or antibodies or antibody fragments. Streptavidin-coated DYNABEADS are commercially available from Dynal AS. Preferably, immobilizing oligonucleotides are produced which bear a biotin moiety which may be used to attach to streptavidin on a solid support.

When detection of target nucleic acid is contemplated, which follows the isolation or capture method according to the invention, at least one of the modules of the modular oligonucleotide may be labelled.

The term "label" as used herein refers to any label which can be assessed qualitatively or quantitatively, directly or indirectly, eg. by virtue of its enzymatic properties, radiation emission, scattering or absorption properties, or of its ability to cooperate with or bind to a complimentary agent to produce a detectable effect, eg. interact with an enzyme to produce a signal, gas evolution, light emission, colour change, turbidity, precipitations etc. Such labels or means for labelling are well known, especially in the field of diagnostic assays and include for example, enzymes, chromophores or fluorophores (eg. dyes such as fluorescein and rhodamine), radiolabels, chemiluminescent compounds or reagents of high electron density such as ferritin, haemocyanin or colloidal gold. A label which uses enzyme activity to generate a colour for spectrophotometric assessment may be employed, for example β-galactosidase, alkaline phosphatase or peroxidase which on the addition of a suitable substrate may generate a signal suitable for detection.

Labels are conveniently introduced into parts of the modular oligonucleotide during or post synthesis. This may be achieved in a similar manner to providing a means for immobilization, by for example providing the oligonucleotide with one partner of a binding pair (pre or post synthesis), and subsequently attaching a second binding partner provided with a label. The first partner may be one of a conventional binding pair, for example biotin:streptavidin or may be part of the oligonucleotide sequence itself to which a second molecule will bind specifically. Alternatively, a derivatized nucleotide bearing a label, for example a radiolabelled nucleotide, may be used in the synthesis of the oligonucleotide or derivatized after synthesis. Alternatively, a module may be synthesized with a portion which is not complementary to the target nucleic acid which may inherently carry a label, e.g. a radiolabel, or be suitable for the attachment of a label. Such extensions to a module are not considered as part of the module when determining if improved binding is observed for a modular oligonucleotide compared to a single oligonucleotide spanning these modules, according to the definition of the invention. It will however be appreciated that generally it will be more convenient if the products to be isolated, ie. the primer extension or sequencing products, are labelled, e.g. by any method described above, in particular by the use of a labelled (e.g. dye) primer for extension or the use of labelled terminator nucleotides. Such labelling is required particularly if the components of the modular oligonucleotides are removed prior to separation of the primer extension products.

For performance of the invention, the method may additionally include the further step of attaching a capture module to a solid support in instances in which the module is provided with means for immobilization, prior or subsequent to the binding of the capture oligonucleotide to target nucleic acid by contacting the sample containing the target molecules with the immobilized capture oligonucleotide. Once bound to a solid support, washing steps may conveniently be performed, especially for purification purposes or to remove background in detection steps. Preferably, the capture oligonucleotide is bound to a solid support prior to the addition of a sample containing target nucleic acid molecules, ie. primer extension products.

Whilst the separate modules may be added separately, in a preferred embodiment, said primer extension products are contacted directly with all modules of said modular oligonucleotide in a single hybridization step.

In procedures employing methods of the invention, additional steps of isolation, separation, purification and/or assessment may be performed as appropriate to obtain the desired results. Thus, for example a method of the invention may comprise at least one of the following additional steps:
a) attaching the capture module of the modular oligonucleotide to a solid support in instances in which the capture module is provided with a means for immobilization;
b) contacting the sample containing the target nucleic acid (primer extension products) with the modular oligonucleotide;
c) contacting the sample containing the target nucleic acid with the modulating modules of the modular oligonucleotide;
d) contacting the sample containing the target nucleic acid with the immobilized capture oligonucleotide to allow binding of the oligonucleotide to target nucleic acid;
e) separating target nucleic acid bound to the capture module from the sample;
f) washing the target nucleic acid separated in (e) above;
g) assessing the presence or amount of label associated with the target nucleic acid after separation of the primer extension/sequencing products.

It will be appreciated that not all of the above steps may be incorporated into any given procedure as for example, steps (c) and (d) essentially perform step (b) in two parts.

The term "assessing" as used herein includes both quantitation in the sense of obtaining an absolute value for the amount of target nucleic acid in a sample, and also obtaining a semi-quantitative or qualitative assessment, for example to indicate simply the presence of target nucleic acid in the sample under study. Assessment may also involve the generation of further molecules for detection, for example by amplification reactions. It will be clear that different sequential steps may be employed to achieve binding of the modular oligonucleotide. For example, a part of the modular oligonucleotide may be contacted with the sample and then bound, followed by contacting and binding of further parts of the modular oligonucleotide. Alternatively the contacting steps may be performed simultaneously.

It will be appreciated that modular oligonucleotides which have utility according to the invention may be made up of different numbers of modules (with or without spaces between them when bound to target nucleic acid molecules), each of which may be different sizes. Whilst the use of modular oligonucleotides directed to different target sites have been shown to have utility (WO98/13522), some appropriate modification of module number and/or module size may be appropriate to obtain optimum binding at a given site under particular experimental conditions. Such optimization is within the scope of the skilled addressee in which trial and error experiments of the type illustrated herein may be employed. Thus, in general, 5 or fewer modules make up the modular oligonucleotide, preferably 2 or 3 modules, with each module containing 4 or more nucleotide bases. Preferably the modular oligonucleotide contains a total of at least 10 nucleotides, preferably at least 18, for example 18, 24, 27, 29, 31, 33 or 36. Modules, when bound to target nucleic acid (ie. primer extension products) are preferably less than 10 nucleotide bases apart, especially preferably less then 2 bases apart, particularly preferably, without any bases in between. It is especially preferred that less than 2 bases separate the capture module and first adjacent modulating module when bound to the target.

Specifically preferred features of modular oligonucleotides for use in the invention are those with 2 or 3 modules, each module with > 5 nucleotides, preferably ≥ 9 ≤ 18, eg. 9, 11, 13, 15 or 18 nucleotides. When 3 modules are employed, slightly shorter modules may be used than when 2 modules are used such that the total nucleotides in 2-part modular oligonucleotides are > 27, preferably ≥ 29, eg. 29, 31, 33 or 36 and in 3-part modular oligonucleotides > 23, preferably ≥ 27, eg. 27, 31, 33 or 36 nucleotides.

The modules of the modular oligonucleotides may be prepared by chemical or other appropriate synthesis well known in the art. Several useful oligonucleotides are available commercially with an attached biotin molecule for immobilization (e.g. KEBO, Stockholm, Sweden).

In a preferred embodiment, a modular oligonucleotide of two modules is employed and one of said modules, the capture module, is immobilized on a solid support.

As mentioned above, in the specific approach the vector-derived sequence(s) as described above is derived from any region of a particular vector and contains a portion which remains intact after cloning of the insert into a particular restriction site of the multiple cloning site of said vector and to which the modular oligonucleotide anneals. Preferably, said portion of the vector to which the modular oligonucleotide anneals is derived from the multiple cloning site or surrounding sequences.

In the generic approach, the vector-derived sequence(s) as described above is derived from any region of a particular vector and contains a portion which remains intact after cloning of the insert regardless of the restriction site of the multiple cloning site of a particular vector into which the insert is cloned, and to which the modular oligonucleotide anneals. Preferably, said portion of the vector to which the modular oligonucleotide anneals is derived from the multiple cloning site or surrounding sequences, especially preferably the sequences preceding, the multiple cloning site. Selection of an appropriate sequence will depend on the vector to be used. In some case the generic approach may not be possible if undisturbed vector-derived sequences are too small. In such case specific approach modular oligonucleotides may be more appropriate.

In general, modular oligonucleotides for use in the isolation of primer extension products have the features as described previously (preferably containing two modules which bind directly adjacently on the target nucleic acid in which modules are between 9 and 18 nucleotides in length) and which are complementary to and capable of binding to the vector-derived sequences of the primer extension products. Particular examples of modular oligonucleotides appropriate for use in the specific or generic approaches described above are listed in Table 1 and comprise preferred features of the invention.

In the case of the specific modular oligonucleotides, as mentioned previously, it is not possible to use all restriction enzyme insertion points although different oligonucleotides can be designed which are appropriate depending on which restriction enzyme is employed. For example the specific pUC18 probes described in Table 1 have utility if the insert is cloned at the BamH1, SmaI or KpnI site. The Bluescript specific modular oligonucleotide in Table 1 may be used when the insert is introduced into any restriction site in the cloning site.

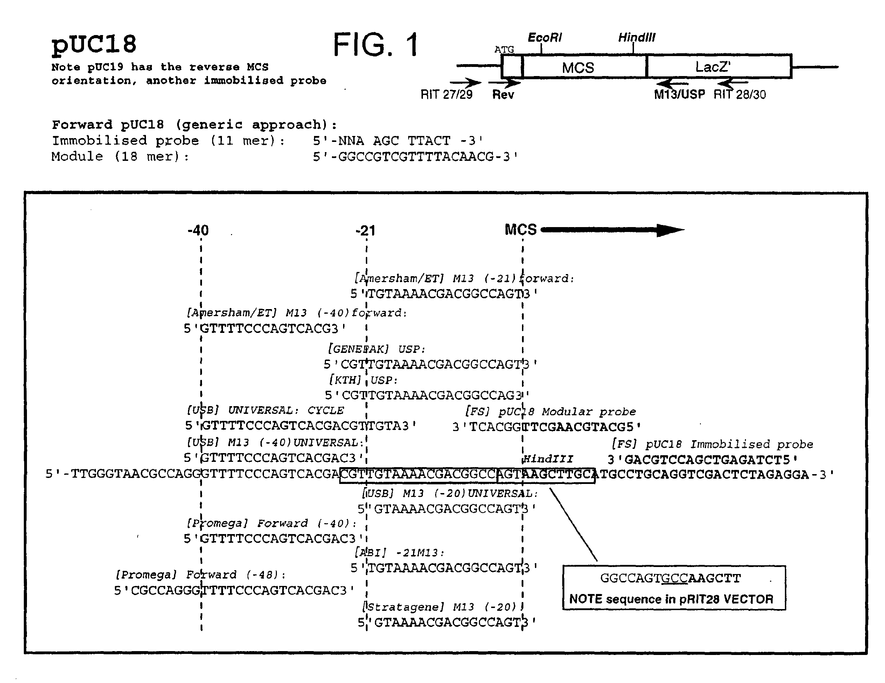

For reference, Figure 1 illustrates the binding site of modular oligonucleotides according to the invention for the vector pUC18 in which the primer extension products containing portions of the vector are generated in the forward direction. The boxed region shows the area of the vector to which the generic approach modular oligonucleotide is directed. In bold, the sequence of a specific approach modular oligonucleotide is given. Suitable primers for the production of primer extension products, such as sequencing products from this vector are indicated.

As used herein, the multiple cloning site of a particular vector refers to those regions known in the art which are present in vectors for insertion of nucleic acid fragments.

It has been found that using the approach described above, ie. using modular oligonucleotides directed to vector-derived sequences, not only is efficient capture of the sequencing products achieved, but also the specificity of the reaction provides improved accuracy in the sequencing reaction (as described in the Examples herein). The results which have been achieved surpass expectation and the capture appears to be achieved without significant non-specific binding. Thus, not only is the method particularly suited to capture of sequencing products for separation, but as a result of the specificity may be used to select whole populations of sequencing products (for a particular insert) from a complex mixture. As illustrated in the examples, different sequencing product populations may be isolated either when these products have been mixed after separate sequencing extension reactions or when different sequencing extension reactions are performed simultaneously.

This has great advantages in situations in which multiplex sequencing is performed since the same primer may be used for the generation of the different sequencing products but the different populations may be selected using modular oligonucleotides directed to unique sequence features of the sequencing product populations (ie. the vector-derived sequences which are different when different vectors are used). This overcomes the disadvantage of using different primers (which may have different efficiencies) and then removing sequences on the basis of the different properties of the primer which has been used.

The above described method of the invention has been optimized and it has been found that optimally hybridization of the capture probe is performed at around 54°C, for example between 40 and 60°C, especially preferably between 50 and 58°C. This was a surprising finding since this value is close to the Tm of the capture probe in the system which was used. It was also found that optimally capture should be performed for at least 15 minutes, for example for 15 to 90 minutes. Furthermore, the amount of the modulating modules used was found to affect the capture efficiency with an optimum of at least 30pmole of the modulating module per 150µg of beads. Thus for example 20 to 50pmoles should be used, for example 30-40pmoles. It will however be appreciated that these values may vary for different systems. Appropriate optimization is well within the scope of the skilled addressee.

It has also been observed, in particular when sequencing products are produced by the use of dye terminator chemistry (e.g. Big Dye Terminator, Perkin Elmer Inc. and ET Terminatory, Amersham Pharmacia Biotech), that the inclusion of PEG (polyethylene glycol), produces a surprising improvement in capturing the products. Thus preferably the method of the present invention comprises binding the modules of the modular oligonucleotide by hybridization in a buffer containing PEG. For example the buffer for capture may contain 6XSSPE (0.9M NaCl, pH 7.4, 60mM Na₂H₂PO₄H₂O, 7.5mM EDTA).

This aspect of the invention also has general applicability for improving hybridization of modular probes to target nucleic acid molecules, particularly products produced by dye terminator chemistry or similar methodology. Thus the present invention also provides a method of improving the binding of a series of consecutive nucleotide bases to a complementary target nucleic acid molecule in a sample, wherein said method comprises at least the step or steps of binding a complementary modular oligonucleotide of at least two parts (modules) including said nucleotide bases to directly adjacent stretches of said target nucleic acid molecule in said sample, wherein said binding step is performed in the presence of polyethylene glycol. Preferably said modular oligonucleotide exhibits improved binding relative to a single oligonucleotide complementary to the region of the target molecule spanned by the modular oligonucleotide, and preferably at least one module (capture module) is immobilized or has means for immobilization.

It has been found that when the method of the invention uses beads as the solid support that the beads may be used repeatedly, e.g. up to 20 additional times by washing the beads in binding/washing buffer after elution of the sequencing products. This thus offers a technique which is readily susceptible to automation. It has also been found that all modules of the modular oligonucleotide are preferably used simultaneously. Thus the present invention provides a one-step method appropriate for use in whole-genome sequencing projects since it offers (i) specific purification of individual sequencing reactions from a multiplex sequencing pool achieved by capture at elevated temperatures and use of modular oligonucleotides, (ii) multiple cycles of reusing the capture beads and (iii) a simple elution protocol using heat and a low salt buffer.

Preferably the method of the invention comprises the steps of:
1) contacting the sample containing the primer extension products with all modules of the modular oligonucleotide, wherein the capture module is immobilized on a solid support;
2) binding said modules by hybridization;
3) separating target primer extension products bound to said solid support;
4) washing said solid support.

The present invention additionally extends to a method of determining the nucleotide sequence of a nucleic acid insert in a vector wherein sequencing products are generated by methods known in the art by performing appropriate extension reactions on said vector, the sequencing products are isolated by the methods described above, the products thus isolated are separated by an appropriate technique, e.g. gel electrophoresis, gas chromatography or HPLC, and the labels carried on said sequencing products are visualized to allow determination of the sequence of said insert or a portion thereof.

In a further aspect, the invention also provides the modular oligonucleotides as described herein and their use in methods of the invention.

The present invention also extends to kits for performing the methods of the invention, comprising at least the following:-
modular oligonucleotides having two or more parts, suitable for use in methods of the invention.

Preferably, at least one of the modules is immobilized on a solid support or has means for immobilization. At least one of the modules may be labelled to allow detection of the target nucleic acid. Additionally, appropriate buffers and/or a solid support may be provided.

The following Examples are given by way of illustration. Further Examples and general methodology relevant to performance of the invention may be found in WO98/13522. The Examples herein contain reference to the following Figures in which:-
Figure 1 shows a schematic representation of modular oligonucleotides for use in isolating primer extension products generated from the vector pUC18 in the forward direction, in which the boxed region shows the area of the vector to which the generic approach modular oligonucleotide is directed, the sequences of a specific approach modular oligonucleotide is given in bold and suitable primers for the production of primer extension products are indicated;
Figure 2 shows a schematic overview of the design of modular and capture probe;
Figure 3 shows the raw data showing the gelfile after gel electrophoresis using as modular oligonucleotides the specific modular oligonucleotide in Table 1 for use with pUC18 in the forward direction. Lane 1-2: capture without modulating module, Lane 3-4: capture with modulating module, Lane 5: ethanol precipitated material;
Figure 4 shows results of optimisations of the modular capture using as modular oligonucleotides the specific modular oligonucleotide in Table 1 for use with pUC18 in the forward direction. (A) titration of the amount of beads, (B) titration of the amount of modulating module, (C) titration of the temperature, (D) titration of the incubation time at 54°C, (E) titration of the incubation time at room temperature;
Figure 5 shows re-use of beads (A) elution with formamide (B) elution with heat and water;
Figure 6 shows specificity and background of the modular capture from multiple cycle sequencing reactions, tested with two bead types, namely A) the specific modular oligonucleotide in Table 1 for use with pUC18 in the forward direction and B) the specific modular oligonucleotide in Table 1 for use with pBluescript in the forward direction;
Figure 7 shows representative and partial chromatograms showing specific capture of individual cycle sequencing reactions from a quatraplex cycle sequencing reaction (two plasmids in two directions, using A) the specific modular oligonucleotide in Table 1 for use with pUC18 in the forward direction, B) the specific modular oligonucleotide in Table 1 for use with pBluescript in the forward direction and C) the generic modular oligonucleotide in Table 1 for use with pBluescript in the reverse direction);
Figure 8 shows a chromatogram showing capture of dye terminator generated sequencing products in the absence of PEG6000;
Figure 9 corresponds to Figure 8 but with the inclusion of 5% PEG6000 during capture of the sequencing products;
Figure 10 shows the effect of loading samples with various amounts of template prepared by ethanol precipitation onto capillaries for electrophoresis by MegaBACE;
Figure 11 corresponds to Figure 10 in which the samples are prepared by capture onto beads using modular probes prior to electrophoresis; and
Figure 12 shows the results of single, duplex or quatraplex sequencing on the PSS robot.

### EXAMPLE 1: Capture of ssDNA using a capture oligonucleotide and at least one additional oligonucleotide as the modular probe.

This example is not an example of the invention, but is provided to illustrate the manyfold increase in the capture of ssDNA by an immobilized capture oligonucleotide when specific oligonucleotide modules have been previously hybridised to the DNA and to describe the methodology used for binding and capture of modular oligonucleotides. The capture oligonucleotide (referred to in this example as capture oligonucleotide or immobilized oligonucleotide) and oligonucleotide hybridized to the DNA (referred to in this example as oligonucleotide modules) together are the modules of the modular probe.

Various combinations of capturing oligonucleotides and oligonucleotide modules for binding to the DNA were used. Thus, 18 mer capture oligonucleotides were used in conjunction with either an 18, 15, 13, 11, 9 or 5 mer oligonucleotide module, 9 mer capturing oligonucleotides with a 9 mer oligonucleotide module with and without a second oligonucleotide module (18, 15, 13, 11, 9 or 5 mer). Also modular probes with a 1 nucleotide space between the annealing sites of the modules were tested. To further investigate the modular effect a 36 mer biotinylated oligonucleotide (comprising of the 18 mer capture oligonucleotide Cl and the 18 mer oligonucleotide module H1-18) was designed and tested for its efficiency in capturing HCV.

### MATERIALS AND METHODS

### PCR amplification (Template generation)

Clones containing the 5' non-translated region (NTR) of two hepatitis C virus (HCV) genotypes (2b and 3a) in the pGEM^{®}-T vectors (Promega, Madison, WI, USA), were used as a template in the PCR to generate a 324 bp fragment for biosensor analysis. PCR amplification was performed with 0.2 µM of each primer OU49 and OD66;
OU49 5'-GGCGACACTCCACCATGAATC-3'
OD66 5'-biotin-GGTGCACGGTCTACGAGACC-3'

Amplification was performed in a 50 µl reaction volume containing 10 mM Tris-HCl (pH 8.3), 50 mM KCl, 2 mM MgCl₂, 0.1 % Tween^{®} 20, 0.2 mM dNTP's and 0.5 U of AmpliTaq^{®} DNA polymerase (Perkin-Elmer, Foster City, CA), using a Perkin-Elmer 9600 thermocycler (Perkin-Elmer, Norwalk, CT). The temperature profile was 94°C for 5 minutes, followed by 30 cycles of (94°C for 15 seconds, 62°C for 45 seconds, 72°C for 60 seconds) and ending with 72°C for 10 minutes.

### Single strand preparation

The biotinylated PCR-products were immobilised onto streptavidin-coated paramagnetic beads (Dynabeads^{®} M-280 Streptavidin; Dynal, Oslo, Norway) and by strand-specific elution a pure template for hybridisation was obtained (Hultman et al., 1989, Nucl. Acids Res., 17, p 4937-4946). Fifty microlitres of PCR-product was captured by incubation for 15 minutes at room temperature with 5 mg/ml of beads in 50 µl binding/washing buffer (10 mM Tris-HCl (pH 7.5) 1 mM EDTA, 2 M NaCl, 1 mM β-mercaptoethanol, 0.1 % Tween^{®} 20). After washing and removal of supernatant, the strands were separated by incubation with 10 µl of 0.1 M NaOH for 5 minutes. The alkaline supernatant with the non-biotinylated strand was neutralised with 6 µl of 0.1667 mM HCl and 1 µl of 280 mM Tris-HCl, pH 7.5, 100 mM MgCl₂. In order to prevent any co-eluted biotinylated strands from interacting with the streptavidin on the sensor chip, a second round of sedimented streptavidin beads (250 µg) was mixed and the supernatant collected. In order to reduce the differences between individual samples within an experiment the eluted single strand DNA was batched. The prepared single stranded DNA was then qualitatively and quantitatively analysed on 10-15% PAGE with PhastSystem^{™} and PhastGel^{®} DNA Buffer Strips and DNA Silver Staining Kit (Pharmacia Biotech, Uppsala, Sweden).

### Oligonucleotides

Biotinylated oligonucleotides for immobilisation onto the sensor chip and oligonucleotides for hybridisation to the ss HCV PCR product were purchased from KEBO, Stockholm, Sweden. The oligonucleotide sequences are shown in Table 2 for location 1 and Table 3 for location 2.

### Biosensor analysis

A BIAcore^{®} 2000 instrument (Pharmacia Biosensor, Uppsala, Sweden) was used in all experiments. Sensor chips SA (Pharmacia Biosensor), precoated with approximately 4000 RU streptavidin (1000 RU corresponds to approximately 1 ng/um² of streptavidin), were used. Experiments were performed at 25°C with 6X SSPE (0.9M NaCl (pH 7.4), 60 mM NaH₂PO₄, 7.5 mM EDTA and 0.005% (v/v) Surfactant P20 (Pharmacia Biosensor)) as injection and running buffer. The biotinylated oligonucleotides for capture onto the sensor chip were immobilised to a level of approximately 500 - 1000 RU (1000 RU corresponds to approximately 1 ng/mm² (Stenberg et al., 1991, J. Colloid Interface Sci., 143, p 513-526) by injection of 50 µl 6X SSPE containing 1 µM biotinylated oligonucleotide at a flow of 30 µl/ minute. Before and after use in hybridisation experiments the sensor chips were treated with three pulses of 50 mM NaOH (5 µl, 5µl/min) to regenerate (R) the surface. One flow cell, without immobilized oligonucleotide was used as reference.

### Hybridisation and single strand capture

Oligonucleotide modules were hybridised to the ss target DNA by incubation in a hybridisation oven for 45 minutes at 54°C with constant rotation then cooled to room temperature. Hybridisation was performed in 100 µl 6x SSPE containing approximately 200 nM ssDNA and 500 nM oligonucleotides.

Forty µl of the hybridisation mix was injected over the immobilised capture oligonucleotide at a flow of 5 µl/min. Controls samples with no hybridizing probes were treated in exactly the same manner. The oligonucleotide surface was regenerated with 50 mM NaOH (5 µl, 5 µl/min).

### RESULTS

A 324 base pair (bp) fragment of the non-translated region (NTR) of HCV was generated by PCR. The dsDNA was melted apart by magnetic bead separation to obtain ssDNA suitable for capture by oligonucleotides immobilised on a sensor chip in the biosensor. This ssDNA was hybridised to oligonucleotide modules prior to injection over the sensor chip which results in a highly efficient capture of the HCV DNA.

In this example biosensor technology was used which allows biological events to be monitored in real time (Jönsson et al., 1991, BioTechniques, 11, 620-672). This method utilises a sensor chip as a solid support for immobilisation. Detection is based on surface plasmon resonance (SPR) to monitor changes in refractive index over time at the sensor surface. The changes are proportional to the mass of molecules bound on the surface and are shown in a so-called sensorgram as resonance units (RU) over time. The immobilisation is rapid and the amount of bound oligonucleotide is determined to be 700 RU by comparing the response units before and after the injection pulse. After regeneration (R) of the sensor surface with 50 mM NaOH, the immobilised capture oligonucleotide was used for capturing the single strand DNA target by hybridisation. When only single strand target DNA is injected over the sensor chip only negligible amounts are hybridised (<20 RU). In contrast, when the single strand target with a preannealed oligonucleotide module H1 (18 mer), designed to be adjacent to the capture oligonucleotide, was passed over the sensor chip (C), significant amounts were retained (250 RU).

### Investigation of the modular effect using an 18 mer oligonucleotide as capture probe in location 1.

An 18 mer biotinylated capture oligonucleotide (C1) was immobilised on a Streptavidin sensor chip. Single stranded HCV DNA with and without previously hybridised oligonucleotide modules was injected over the sensor chip as described in the methods section. The results represent normalised values from independent experiments as a consequence of the variation of absolute responses between different sensor chips, depending on the amount of streptavidin coated onto the chip surface, thus affecting the amount of immobilised capture oligonucleotide and capture efficiency. Low capture efficiency was observed with ss DNA injected over an 18 mer immobilised capture oligonucleotide (C1). A similar low capture was also achieved even if a 5' spacer of 10 adenines was used with the 18 mer (C1x10A, Table 2). In the subsequent capture experiments a prehybridised complex comprising of ss target DNA and oligonucleotide modules was used. The oligonucleotide modules varied from 18 to 5 nucleotides in length, but all were designed to anneal adjacent to the 3'-end of the immobilised capture oligonucleotide (Table 2). Using the specific oligonucleotide modules (H1-18, 15, 13, 11; 18 to 11 mers) a significant increase of capture (230 to 320 RUs) was observed, as compared with the experiment lacking an oligonucleotide module. However, the capture efficiency was greatly diminished and sometimes abolished when H1 was less than 9 nucleotides in length. The different controls clearly indicated the specificity on hybridisation between the immobilised capture oligonucleotide and the single strand DNA/ oligonucleotide module complex as the responses arising from interactions of oligonucleotides or nonspecific DNA were negligible. No capture occurred when an 18 nucleotide gap was present between the different modules.

### Investigation of the modular effect using a 9 mer oligonucleotide as capture probe.

A 9 mer biotinylated capture oligonucleotide (C2) was immobilised on a SA sensor chip and the oligonucleotide modules fractionated into smaller modules. As expected no capture of DNA was observed when an immobilised capture nonamer (C2) was used alone. When the nonamer oligonucleotide module (H4) and the oligonucleotide module (H1 - 18, 15, 13, 11, 9) were used, ss DNA was successfully captured. However, module assisted capture with the short pentamer module (H1-5) was unsuccessful as well as use of a single nonamer oligonucleotide module (H4). These results suggest that it is not the length of the capture oligonucleotide that is the most important parameter, rather it depends on the number and length of oligonucleotide modules that are employed. Control experiments were carried out both for the 18 and 9 mer immobilised capture oligonucleotides to verify the concept. Firstly, non-specific interactions were investigated by injecting a similar sized non-specific DNA over the capture oligonucleotide and by injecting the oligonucleotide module along. No increase in response was observed. Secondly, non-specific DNA was co-incubated with the target DNA and the oligonucleotide module. Capture was still specific and no interference from the non-specific DNA was observed. Hence the modular approach has the ability to capture a specific target without any reduction in signal when challenged with unrelated DNA. No capture occurs when an 18 nucleotide gap is present between the non-capture oligonucleotide modules.

### The effect of gaps between oligonucleotide modules

From the previous experiments the detrimental effects on capture efficiency with 18 nucleotide long gaps between oligonucleotide modules was clear and/or immobilised capture probe. To further analyse the restrictions in the oligonucleotide module assisted capture approach, single nucleotide gaps were introduced between the modules of the modular probe. The H1-11 mer capture oligonucleotide and the two nonamer oligonucleotide modules (H4 and H1-9) were reconstructed and their annealing sites shifted one nucleotide towards the 5' end of the target DNA and were renamed H2, H5 and H3 respectively (Table 2). Comparison showed that discontinuous probes were able to capture single strand target, although at a lower efficiency.

### Fragmentation of long oligonucleotides into shorter modules

This experiment was performed to further investigate whether the efficiency could be improved by fragmentation of extended capture oligonucleotides into shorter modular units. When an immobilized 27 mer oligonucleotide was used for capturing or when two oligonucleotides with a total annealing length of 27 nucleotides were used, the capture of DNA was poor. However when this 27 nucleotide stretch was fragmented into three nonamers a highly efficient capture was observed. This was further substantiated by a 36 nucleotide capture oligonucleotide (C4) (Table 2) which fails to efficiently capture single strand target DNA, while use of oligonucleotide modules and a shorter immobilised capture probe significantly improves the capture.

To investigate whether this effect is observed at another position in the HCV genome (location 2), a second 18 mer capturing oligonucleotide was designed together with an 18 mer oligonucleotide module. The oligonucleotide sequences are shown in Table 3.

### Investigation of the modular effect in location 2 of the HCV genome.

An 18 mer biotinylated capture oligonucleotide (OMD2) was immobilised on a SA sensor chip as described above. Injection of ssDNA with an 18 mer oligonucleotide module (OMD6) resulted in an increase of 400 RU while ssHCV alone was not captured.

### CONCLUSIONS

This example illustrates that ssHCV is captured poorly or not at all by the 18 mer oligonucleotide immobilised on the chip. When the ssHCV was incubated with an oligonucleotide module of 18, 15, 13 or 11 nucleotides a significant increase in capture was observed. However incubation of ssHCV DNA with a 9 mer or 5 mer oligonucleotide module did not result in an increase in capture. When a 36 mer capture oligonucleotide was used in place of the 18 mer capture oligonucleotide and the 18 mer oligonucleotide module little capture of HCV was observed. These results further substantiate the modular effect. Non-specific hybridisation of the HCV DNA was investigated by injecting a similar sized non-HCV ss PCR product (treated in the same manner) over the capture oligonucleotide. Non-specific hybridisation of the oligonucleotide modules was investigated and was also ruled out.

No capture of DNA was observed when a 9 mer immobilised capture oligonucleotide was used alone for capture or with a single 9 mer oligonucleotide module. However, when two 9 mer oligonucleotide modules were incubated with the DNA, a highly efficient capture was observed by the immobilised 9 mer. These oligonucleotides anneal at the same position on the DNA as the 18 mer capturing oligonucleotide with the 9 mer oligonucleotide module but hybridisation is only observed when 2 oligonucleotide modules are used as opposed to 1 oligonucleotide module.

When an 18 and a 9 nucleotide space between the second and third oligonucleotides of the modular probes was inserted the modular effect was abolished. To further investigate the effect of inserting a space between the modules, three 9 mer oligonucleotides with a one nucleotide gap between them were designed. These one nucleotide gaps do seem to result in slightly less capture of HCV DNA but nevertheless good hybridisation signals of approximately 50 to 100 RU were obtained. The gap between the capturing oligonucleotide and the first oligonucleotide module resulted in slightly more efficient capture than the gap between the second and third modules. A single gap between the modules of a modular probe with 2 modules also resulted in improved capture over the non-modular probe, but was reduced with respect to a 2-module modular probe without a gap between modules.

The results for experiments using modular probes complimentary to location 2 further support the modular theory, as a 200 fold increase in signal was observed when the 18 mer oligonucleotide module was hybridised to the DNA prior to capture by the immobilised oligonucleotide.

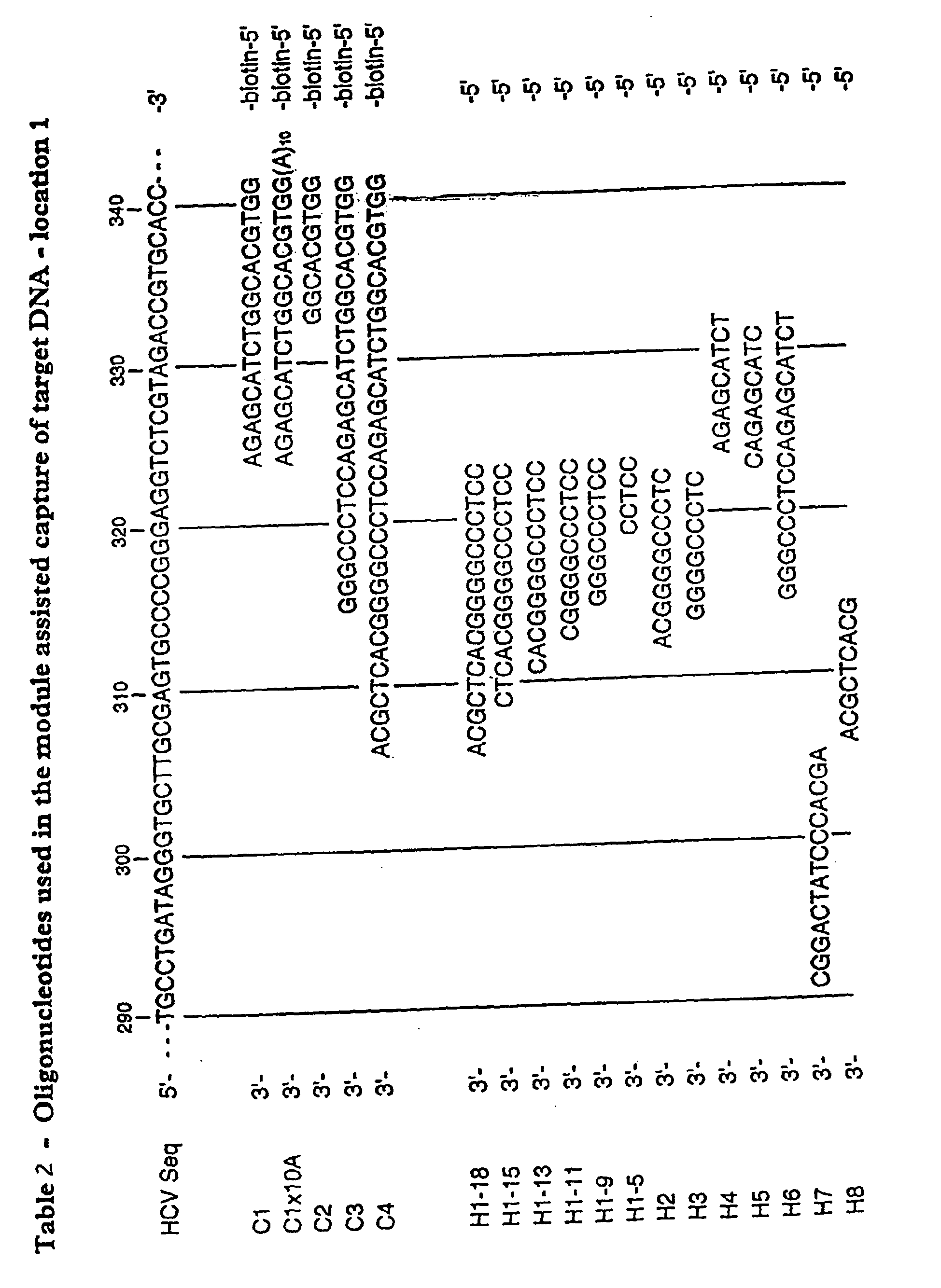

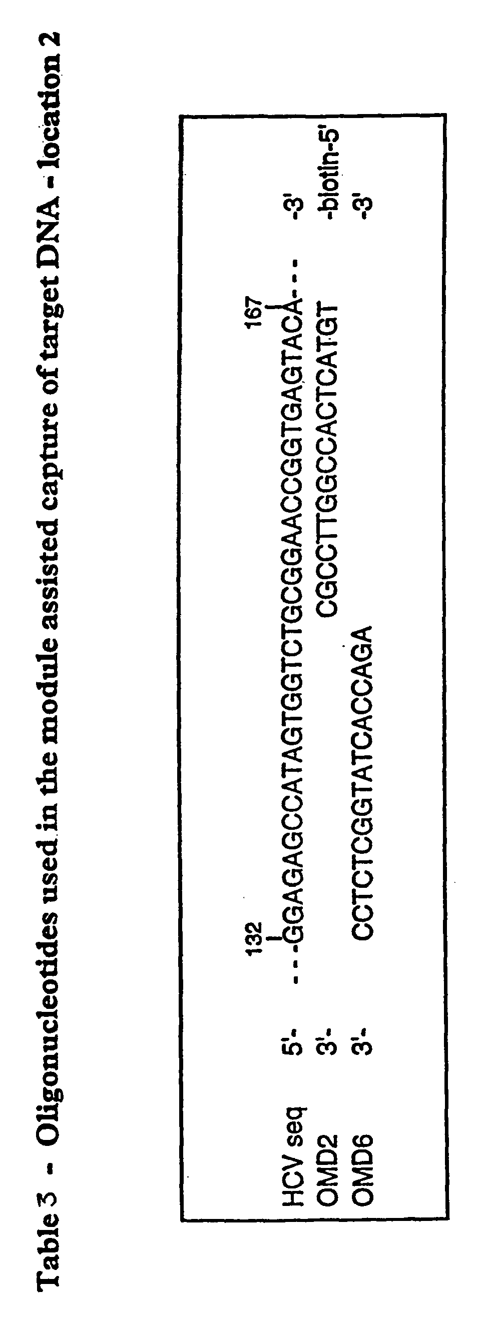

### EXAMPLE 2: Capture of sequencing products generated by the USP primer using a capture oligonucleotide and at least one additional oligonucleotide as the modular probe.

Methods for the identification and/or capture of sequencing products generated by extension of the universal sequencing primer (USP) are performed analogously to those described in Example 1 for HCV using the following modular probes:
JL-H1/USP + JL-C2/USP (9+9)
   3'-GACGTCCAG-5' + 3'-CTGAGATCT-5'-biotin, or
JL-H2/USP + JL-C1/USP (13+18)
   3'-GTTCGAACGTACG-5' + 3'-GACGTCCAGCTGAGATCT-5'-biotin, or
JL-H2/USP + JL-H1/USP + JL-C2/USP (13+9+9)
   3'-GTTCGAACGTACG-5' + 3'-GACGTCCAG-5' + 3'-CTGAGATCT-5'-biotin
in which JL-C1/USP + JL-C2/USP are capture oligonucleotides.

### EXAMPLE 3 Use of modular oligonucleotides to capture sequencing products

The following example describes the use of modular oligonucleotides to capture the population of sequencing products corresponding to a particular primer/vector/insert which may optionally be present in a complex mixture with other populations of sequencing products.

### MATERIALS AND METHODS

### Single template experiments

### Preparation of PCR-products

pUC18 and pBluescript plasmids containing various inserts were used as a template in PCR with RIT 27 (5'-GCTTCCGGCTCGTATGTTGTGTG-3') and RIT 28 (5'-AAAGGGGGATGTGCTGCAAGGCG-3') primers. Amplification was performed in a 50 µl reaction volume containing 10 mM Tris-HCl (pH 8.3), 50 mM KCl, 2 mM MgCl₂, 0.1 % Tween^{®} 20, 0.2 mM dNTP's, 0.5 U of Taq DNA polymerase (Perkin-Elmer, Norwalk, CT) and 0.075 µM of each primer, using a Perkin-Elmer 9600 thermocycler (Perkin-Elmer, Norwalk, CT). The temperature profile was 94°C for 5 minutes, followed by 30 cycles of 94°C for 30 seconds and 72°C for 2 minutes, and ending with 72°C for 10 minutes.

### Cycle sequencing with four colour dye primer

The following reagents were mixed together (on ice): A and C mix: 26 mM Tris-HCl (pH 9.0), 6.5 mM MgCl₂, 150 µm dNTP, 0.5 µm ddNTP (A or C), 1.5 U ThermoSequenase (Amersham Pharmacia Biotech, Sweden), 20 nM Universal (forward) sequencing primer (USP), 5'-dye-CGTTGTAAAACGACGGCCAGT-3', or Reverse sequencing primer (RSP), 5'-dye-TTCACACAGGAAACAGCTATGACC-3', A reactions Joe-dye and C reactions Fam-dye (Genpak Ltd, England), 1 µl PCR product and H₂O to a final volume of 10 µl per reaction.
G and T mix: 26 mM Tris-HCl (pH 9.0), 6.5 mM MgCl₂, 150 µm dNTP, 0.5 µm ddNTP (G or T), 1.5 U ThermoSequenase, 20 nM USP or RSP primer, G reactions Tamra-dye and T reactions Rox-dye (Genpak Ltd, England), 2 µl PCR product and H₂O to a final volume of 20 µl per reaction.

Cycle sequencing was carried out using a temperature profile of 95 °C for 1 minute followed by 28 cycles cycling between 96 °C for 30 seconds and 56 °C for 1 minute. The cycle sequencing products were subsequently pooled prior to solid phase capture.

### Preparation of beads

Fifteen microlitres of paramagnetic beads (10mg/ml), covalently coupled to a capture probe (selected from Table 1), were washed twice in 15 µl binding/washing (B/W) buffer (10 mM Tris-HCl pH 7.5, 1 mM EDTA, 2M NaCl, 1mM β-mercaptoethanol, 0.1 % Tween 20). The washed beads were resuspended in 15 µl 6XSSPE (0.9 M NaCl pH 7.4, 60 mM NaH₂PO₄H₂O, 7.5 mM EDTA).

### Capture of cycle sequencing products

Fifty-five microlitres of the pooled cycle sequencing product was incubated with 30 pmoles of modulating module (selected from Table 1), 150 µg beads and H₂O in 115 µl 6XSSPE at 54 °C for 15 minutes. The beads were washed once in 100 µl B/W buffer and once in 100 µl 1 X TE buffer. The captured cycle sequencing products were eluted from the beads with 2 µl of 95% formamide prior to loading on a 377 ABI sequencer.

### Optimization

The assay was optimized for the specific pUC18 forward bead (Table 1). The amount of beads was varied between 40 µg and 250 µg, which resulted in a change in total volume between 104 µl and 125 µl and the efficiency was determined by analysis of the automatically generated peak signals for the different fluorophores after each gel electrophoresis experiment. Next the amount of modulating module was varied between 0 and 50 pmoles using 150µg of beads (in a constant total volume of 115 µl). A temperature titration between 20° and 70° was performed using 150µg of beads and 30pmole modulating module, concentrating on the temperature interval close to the annealing temperature of the immobilised probe. Finally incubation times, both at 54°C and following incubation at room temperature, between 0 and 15 minutes were studied.

### Sequence product mixtures

Experiments with mixtures of different cycle sequencing products were performed in order to study the specificity of the capture probes. To further investigate the specificity pUC18 beads were incubated with pBluescript cycle sequencing products and vice versa. To achieve this a bead with an immobilised probe specific for pBluescript was synthesised together with a suitable modulating module (Table 1). PCR products for these two templates were generated and subsequently used for 4 separate cycle sequencing reactions in both directions using a forward and reverse sequencing primer, identical for the two vectors. These four cycle sequencing products were then mixed into equal proportions to either 55 µl (normal volume for single sample) or 220 µl (4 times the volume for a single sample) final volume mixtures. The capture beads were then used to enrich for its specific sequencing product, i.e the pUC18 bead captured sequencing reactions generated by the forward sequencing primer on pUC18 vector and the pBluescript bead captured sequencing reactions generated by the forward sequencing primer on pBluescript vector. Capture was performed as usual for the smaller volume but when 220 µl were used the total volume of the reaction was increased to 354 µl.

### Elution of captured sequencing products using water and heat

The cycle sequencing products were eluted from the beads either with 2 µl of 95% formamide as described above, or with water and heat. In the latter case captured cycle sequencing products were resuspended in 10 µl water and heated to 95° C for 3 minutes. The supernatant was transferred to new tubes while still hot. Loading buffer (2 µl of 95% formamide) was added to the supernatant prior to evaporation of the water at 95° C.

### Re-use of beads

Experiments with re-use of beads were performed with both elution methods. Six iterative captures were done using the same beads. Between captures the beads were washed in the same manner as described under preparation of beads.

### Comparative ethanol precipitation

Fifty-five microlitres of the cycle sequencing product was precipitated with 138 µl of 96% ethanol and 5.5 µl of 3 M NaAc and, after incubation at -20°C for 10 minutes, the DNA was recovered by centrifugation for 20 minutes. The resulting pellet was washed in 700 µl 80 % ethanol followed by centrifugation for 5-10 minutes. Finally, the pellet was dried (over night or in speedvac for 10 minutes) and resuspended in 2 µl 95 % formamide.

### Multiplex experiments

Multiplex cycle sequencing with four colour dye-primer PCR products, obtained as described above, from both pUC18 and pBluescript were used as templates in a quatraplex cycle sequencing reaction. In order to obtain four different sequencing products both universal (USP) and reverse (RSP) sequencing primer were used. The reaction was carried out as described above for a single template with the amount of water reduced to compensate the increased volumes of primer and PCR product.

### Capture of cycle sequencing products from multiplex cycle sequencing

In the quatraplex cycle sequencing described above four different sequencing products are obtained. These were captured in an iterative fashion with their respective bead and modulating module. After incubation with the first bead the supernatant was transferred to a new tube and 150 µg of the next bead and 30 pmol of the corresponding modulating module were added. Another incubation of 15 minutes at 54 °C was performed. This was repeated for each sequence. The beads with captured sequencing products were treated as described for a single template.

### RESULTS

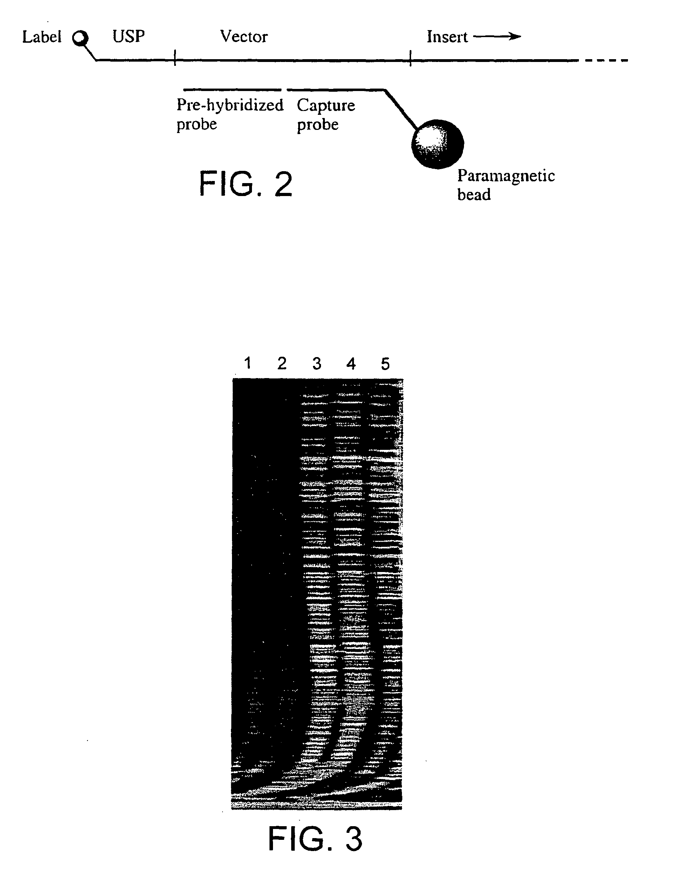

In these experiments, a model system consisting of a pUC18 plasmid target with an insert of approximately 1200 bp and a capture bead with an immobilised probe (Table 1) complementary to a common vector-derived sequence of the generated Sanger fragments together with an adjacent positioned modulating module (Table 1, Figure 2) was used. DNA templates, in this case PCR products, were used as DNA source in a cycle sequencing reaction resulting in single-stranded Sanger fragments. These fragments were captured onto the bead surface by hybridisation in a one-step reaction by mixing with beads (with an immobilized probe) and a modulating module.

In the initial experiments with the model system the beads with captured fragments were washed and then resuspended in formamide (by which the fragments were eluted from the bead surface) and loaded on an automated DNA sequencer.

An example of these initial experiments is shown Figure 3 which displays the corresponding gelfile after a capture performed at 54°C. Indeed this initial and relatively quantitative analysis showed the advantage of an assisting modulating module leading to similar fragment intensities as the reference sample (lanes 3 and 4 compared to lane 5). In addition, the quality of the different obtained DNA sequences were compared using the standard evaluation software indicating improved accuracy using the capture strategy (data not shown). The ethanol precipitated material had an accuracy of 92.6% over the 500 first bp, while capture with modular probe had an accuracy of 96.6%. The accuracy for samples without probe was 90.2%.

### Optimisations of capture efficiency

To further improve the capture protocol a number of parameters were investigated such as the amount of beads and modulating module, incubation temperatures and time.

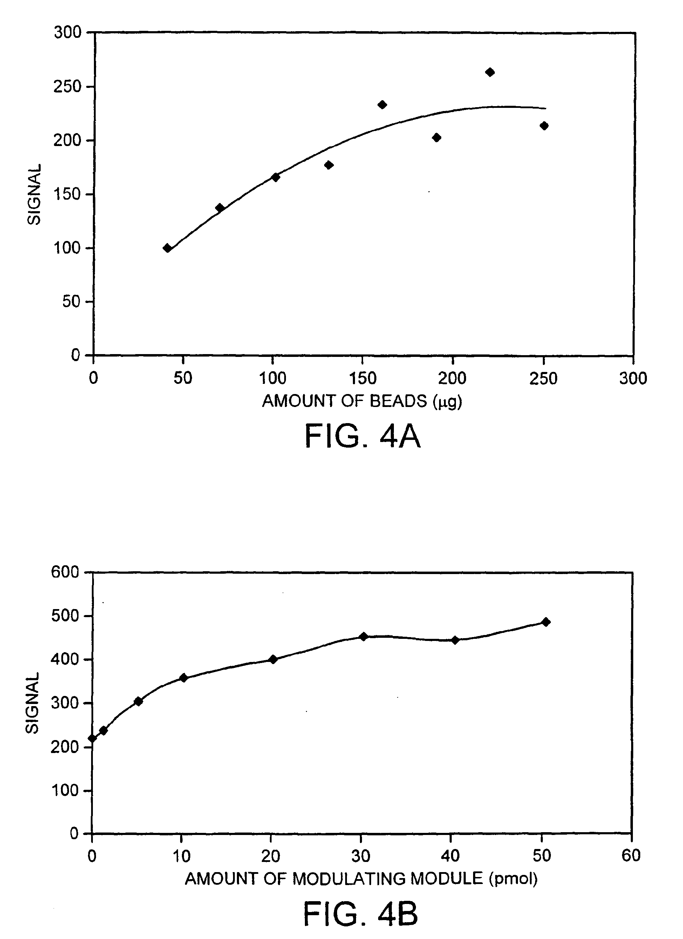

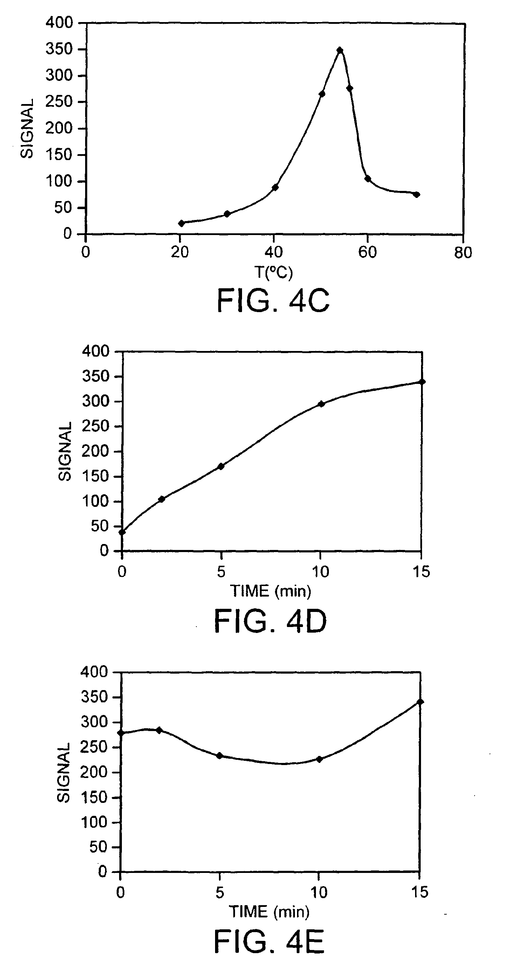

Again the model system consisted of the pUC18 system and the corresponding capture bead. In the first set of experiments the amount of beads were investigated. Triplicate samples (in all optimisations) were analysed in the range of 40 to 250 µg beads. As shown in Figure 4A a slight decline in efficiency is observed for lower amounts than 150 µg beads. Secondly, the amount of modulating modules were investigated in the interval 0 to 50 pmol per capture. A decline in efficiency was observed for lower amounts than 30 pmoles (Figure 4B). Thirdly the capture temperature was investigated in the interval 20°C to 70°C. As demonstrated in Figure 4C the capture temperature has a clear effect on the efficiency with a maximum yield at 54°C (close to the Tm of the immobilised probe) and a rather sharp declining curve at both higher and lower temperatures. Finally the incubation time at the optimum capture temperature (54°C) as well as the subsequent incubation at room temperature was investigated. From the data presented in Figure 4D and 4E it is clear that the incubation at the capture temperature requires at least 15 minutes in order to achieve a maximum yield in capture, while the yield is rather invariant in respect to incubation time at room temperature.

### Re-use of beads

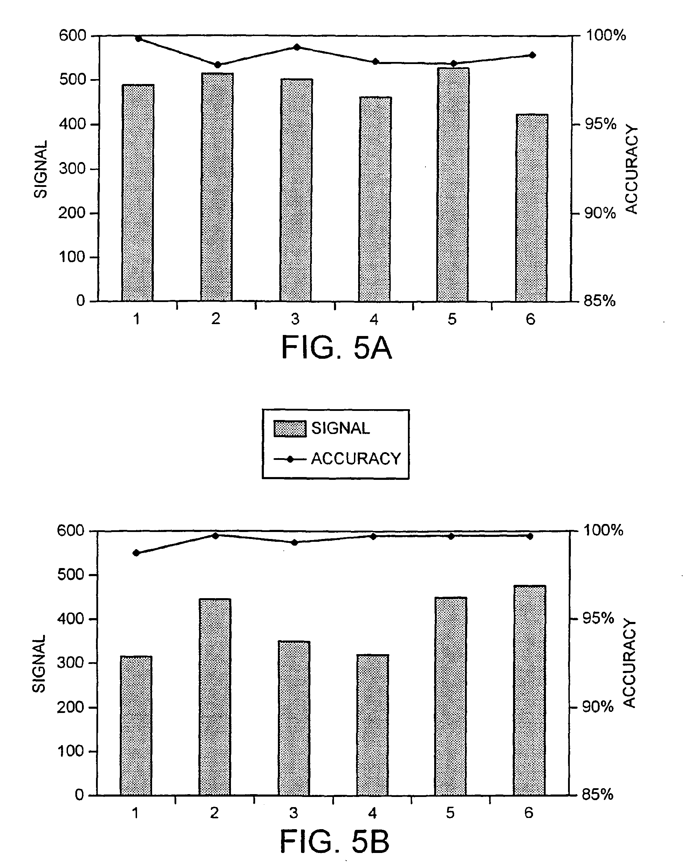

Two formats for iterative re-use of beads were investigated. The first protocol is based on formamide elution after capture and direct loading onto the gel, while the second protocol involves a heat release at 95°C in a low salt buffer and transfer of the supernatant into a new tube to which formamide is added. Remaining water is evaporated by heat. The results for the first protocol is shown in Figure 5A for 6 iterative cycles of re-use. The graph presents signal intensities and the accuracy over the 500 first bases after each cycle. The signal and the accuracy remains high even after 6 cycles. The corresponding data for the heat release, the preferred protocol from the point of automation and handling of toxic formamide, demonstrate an even higher accuracy (Figure 5B).

### Specificity of the capture protocol

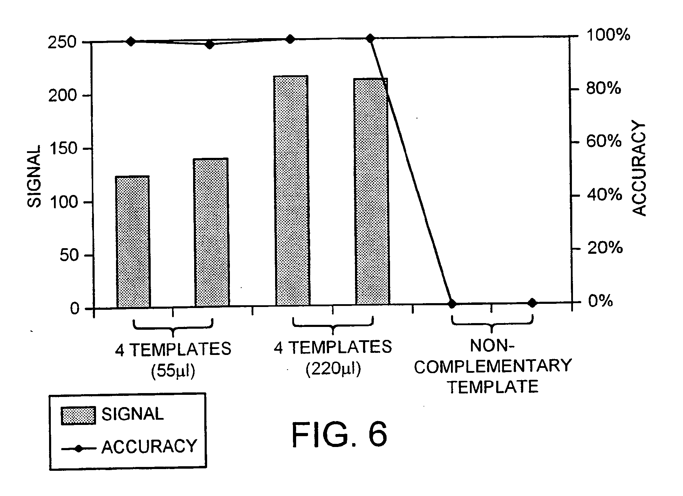

In order to investigate the specificity of the optimised protocol we extended the model system to involve an additional plasmid vector, pBluescript with an insert of approximately the same length as the previously used pUC18 plasmid. The method is described under "Sequence product mixtures" above in which the products of 4 separate cycle sequencing reactions were mixed and capture of selected populations was performed. The data which were obtained is shown in Figure 6. Each bar corresponds to use of a specific bead and it is evident that specific capture is achieved with excellent accuracy over 500 bp and as expected higher signal are generated by increasing the amount of sample. As a control a non-related cycle sequencing product with no homology with the immobilised probe or modulating module was also tested with the two bead types giving no measurable signal (Figure 6).

### Multiplex sequencing and purification

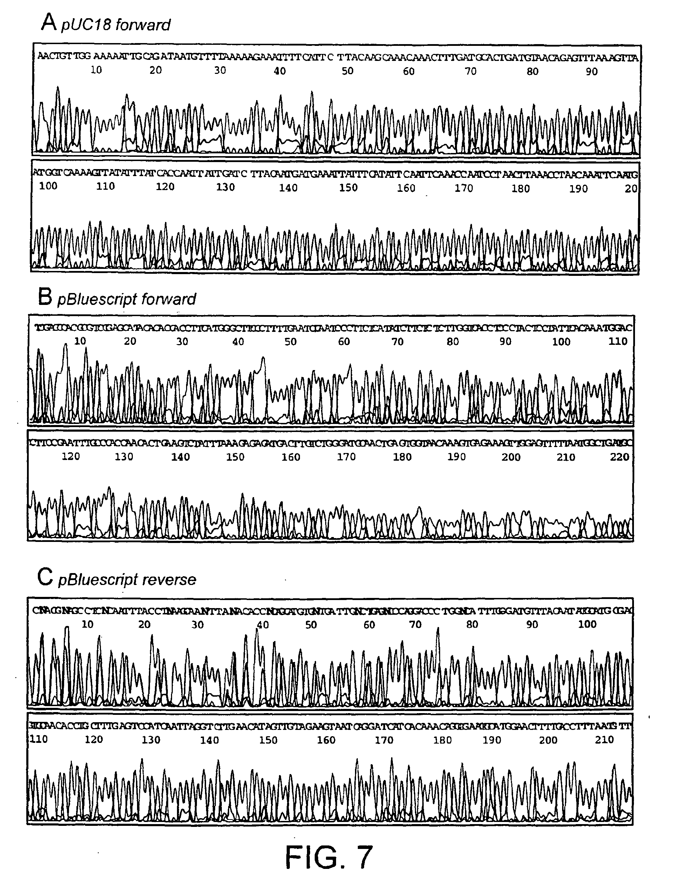

A multiplex system was established to take advantage of the unique specificity obtained with the capture beads and accompanying modulating module. The principle was to run parallel cycle sequencing reactions in a single tube corresponding to forward and reverse sequencing primer with two plasmids as targets (containing inserts of approximately 800 to 1500bp) and from the generated pool of various sequencing reactions iteratively enrich individual reactions with the use of capture beads. For example in the first round of capture the pUC18[reverse] beads are used to capture the corresponding targets, and the supernatant is subsequently moved to the next step, capture with for example pUC18 [forward] beads etc. This then results in four beads with "directionally" captured material which enables individual elution and loading. Representative chromatograms are shown in Figure 7 demonstrating specific capture of three of the individual cycle sequencing reactions from a quatraplex cycle sequencing reaction (two plasmids in two directions).

### DISCUSSION

The above results show that purification of cycle sequencing products by ethanol precipitation may be replaced with a capture assay using modular oligonucleotides.

### EXAMPLE 4 Use of PEG during hybridization, use of modular probes in capillary electrophoresis and multiplex sequencing

### MATERIALS AND METHODS

### DNA samples (plasmids and PCR products)

pUC18 and pBluescript plasmids containing various inserts (from bacterial genome shotgun libraries) were used either directly in sequencing reactions (see below) or as template in a PCR amplification with RIT27 (5'-GCTTCCGGCTCGTATGTTGTGTG-3') and RIT28 (5'-AAAGGGGGATGTGCTGCAAGGCG-3') primers as described in Example 3 using AmpliTaq DNA polymerase (Perkin-Elmer, Norwalk, CT).

### Cycle sequencing with dye primers and dye terminators.

The dye primer cycle sequencing reactions for the Amersham Pharmacia Biotech MegaBACE 1000 sequencer (MegaBACE) were performed as described in the ET primer kit provided by Amersham Pharmacia Biotech (Cleveland, USA). The dye terminator cycle sequencing reactions for the Applied Biosystem 377 XL Prism DNA sequencer (ABI 377) were performed as described in the Big Dye Terminator kit provided by Perkin Elmer, Norwalk, Connecticut, USA. Multiplex sequencing was performed by combining two primers (USP and RSP) and/or two different samples (PCR products) from random pUC18 and pBluescript clones.

### Capture and elution of cycle sequencing products

The cycle sequencing products were incubated as described in Example 3. Briefly, the products were incubated with 30 pmoles of modular probe and the prewashed beads (150 µg) in a total volume of 115 µl 6XSSPE (0.9 M NaCl pH 7.4, 60 mM NaH₂PO₄ H₂O, 7.5 mM EDTA) at 54 °C for 15 minutes. In the case of multiplex sequencing the supernatant was transferred to a new tube with new beads for iterative capture. The beads were then washed once in 100 µl B/W buffer (10 mM Tris-HCl pH 7.5, 1 mM EDTA, 2 M NaCl, 0.1 % Tween 20) and once in 100 µl 1 X TE buffer. The captured cycle sequencing products were then eluted from the beads at room temperature with 2.5 µl of loading buffer (PE Applied Biosystems, UK) prior to loading onto the ABI 377 XL Prism DNA Sequencer or with 10 µl of loading buffer (Amersham Pharmacia Biotech) prior to injection onto MegaBACE DNA sequencer. For capturing of sequencing products (both singleplex and multiplex reactions) generated with Big Dye terminator chemistry the capturing buffer comprised also of PEG6000 (polyethylene glycol) (Merck) added to a final concentration of 5%.

### Results

### (A) Use of PEG6000 for capturing dye terminator generated sequencing products

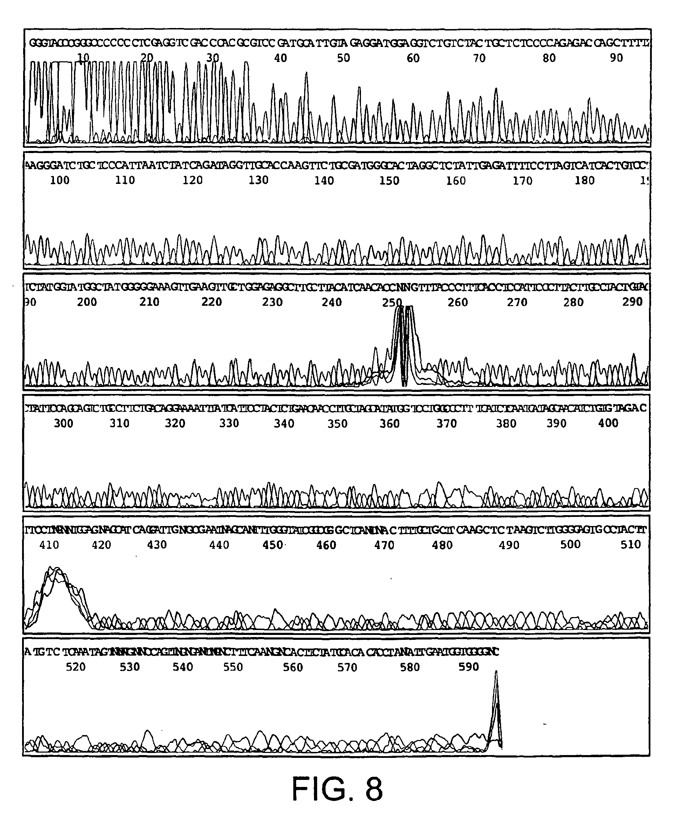

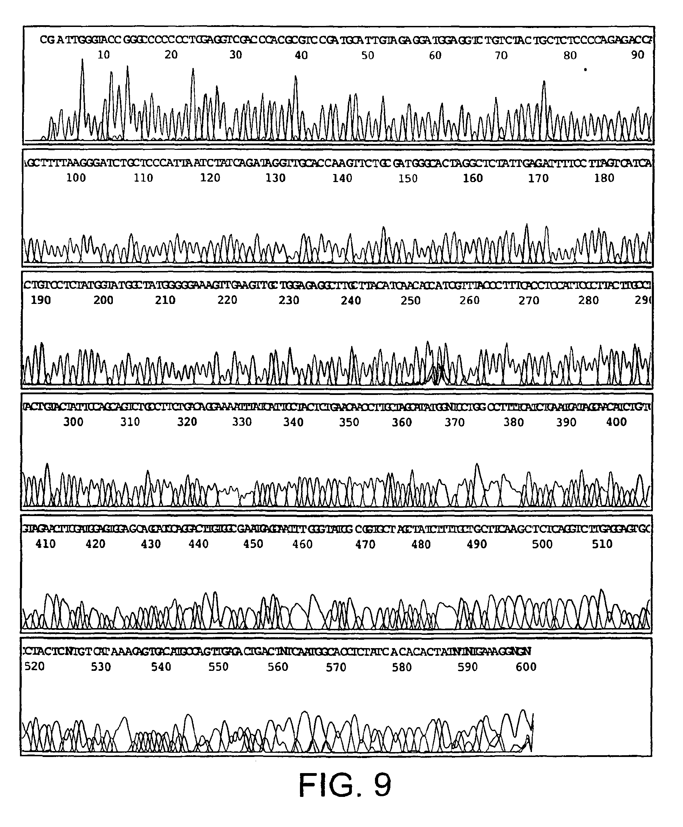

A pBluescript clone was amplified by PCR using vector primers and was subsequently sequenced using a non-labelled universal sequencing primer according the manufacturer. The capture using pBluescript forward beads and corresponding modular probe were carried using different amounts of PEG6000 in the capturing buffer. Washing and elution was performed as previously described and the sequencing products were loaded onto a ABI377 DNA sequencer. Optimal binding was obtained with 5 % PEG6000 as determined by analysis of the ABI gelfile (data not shown). The corresponding chromatograms for 0% and 5% PEG6000 are shown in figure 8 and figure 9. The capturing buffer without PEG (fig 8) shows weaker signal (varying between 45 to 162 relative units), shorter read length and higher background to the capture with 5% PEG having high signals (269 to 803 relative units), long read length and more even signals.

### (B) The effect of different amounts of plasmid template in capillary electrophoresis

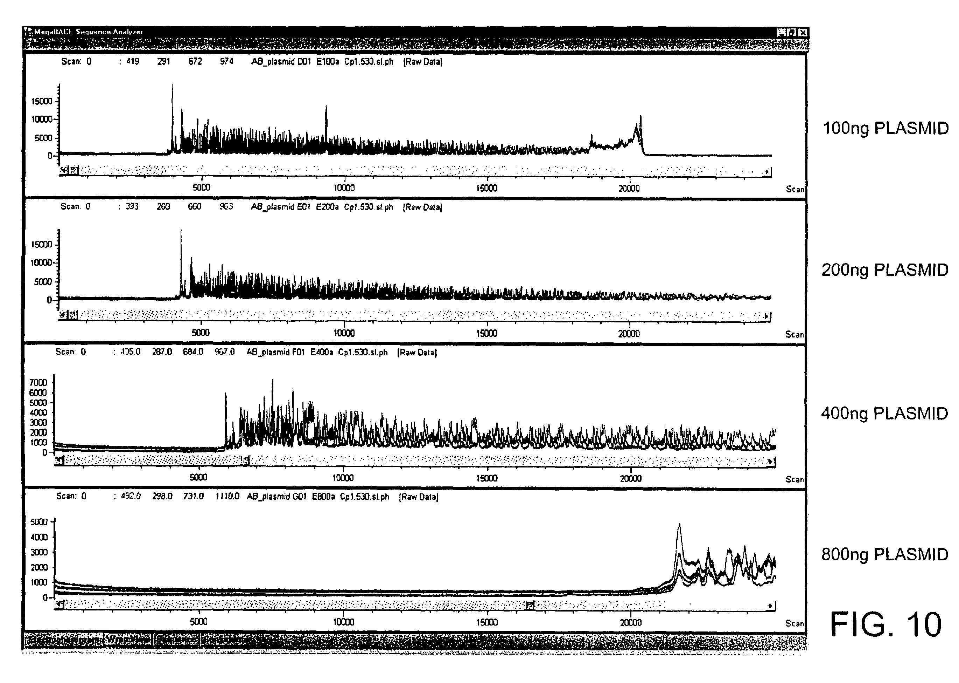

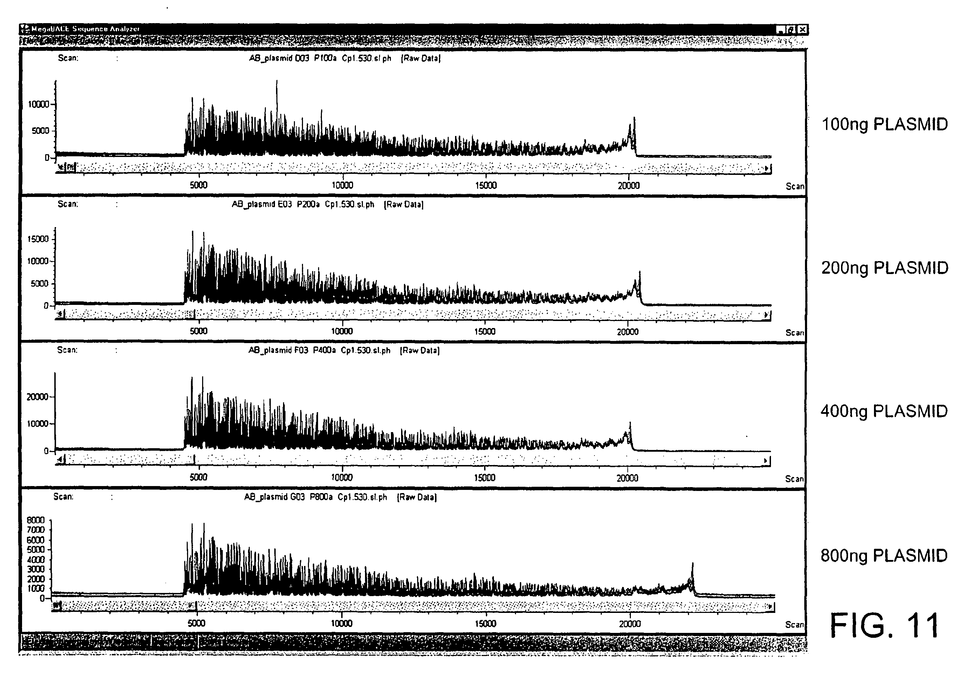

It is a well known fact that the capillary electrophoresis suffers from limitations when too high amounts of template DNA are used during the electroinjection onto the capillary. This is exemplified in figure 10 (chromatogram overview) and Table 4 (summary) which shows the results after performing cycle sequencing reactions with dye primer chemistry (Mega BACE, Amersham Pharmacia Biotech) using different amounts of pUC18 plasmid (25 ng to 1200 ng plasmid) and then subsequently precipitated by ethanol. As shown in figure 10 for 100 to 800 ng plasmid, the sequencing peaks are delayed when increasing amounts of plasmid are used resulting in no readable sequence at the highest concentrations. In contrast, figure 11 (chromatogram view), Table 4 (summary), different amounts of plasmid could be used given long sequencing reads even for high concentrations of plasmid when the magnetic bead capture was used. Thus this experiment clearly shows that the problems with high concentrations are eliminated with the bead approach.

### (C) Efficiency of multiplex sequencing using capillary electrophoresis.

In order to further evaluate the efficiency in multiplex sequencing using capillary electrophoresis in an automated environment an experiment was designed based on pooling two PCR products into a common cycle sequencing reaction. This was achieved by PCR amplification of eight pUC18 random clones and eight pBluescript random clones and then pooling of one each product into a common tube for cycle sequencing using dye primer chemistry (MegaBACE, Amersham Pharmacia Biotech). Thus the cycle sequencing simultaneously generated four sequencing products. Phrep20 is an established algorithm that determines how far one can read (number of bases) and MB is a similar algorithm developed for the instrument.

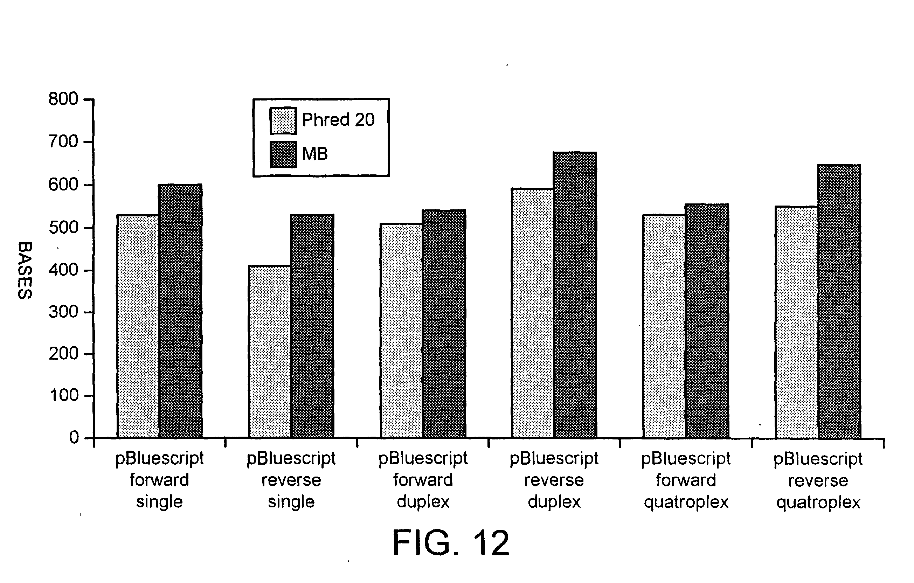

Figure 12 shows the iterative magnetic pullout of the sequencing reactions generated with pBluescript forward bead (and modular) (first capture) and then the supernatant was incubated with pBluescript reverse bead (and modular) (second capture) for the respective pullout. Thus after this, two sequencing reactions remain in the supernatant. In addition these manipulations have been achieved using an in-house robotic instrument, PSS (Anders Holmberg, unpublished) ruling out manual mistakes/variation. As a comparison both the corresponding single reactions (one template and one sequencing primer) and duplex reaction (one template and two sequencing primers) are included. From this data it is clear that the read length is kept despite the multiplexing using any of two alternative basecalling algorithms.

**Table 4: Effect of template concentration on sample prepared by ethanol precipitation versus capture onto beads with modular oligonucleotide - summary**

| **Ethanol precipitation** | | |
|---|---|---|
| *ng plasmid* | *Scan time* | *Basecall* |
| 25 | 4 | 677 |
| 50 | 4 | 672 |
| 100 | 4 | 689 |
| 200 | 4 | 640 |
| 400 | 6 | 566 |
| 800 | 21 | 4 |
| 1200 | 16 | 56 |

| **Magnetic beads** | | |
|---|---|---|
| *ng plasmid* | *Scan time* | *Basecall* |
| 25 | 4 | 690 |
| 50 | 4 | 693 |
| 100 | 4 | 706 |
| 200 | 4 | 744 |
| 400 | 4 | 695 |
| 800 | 4 | 689 |
| 1200 | 4 | 728 |

## Claims

1. A method for capturing primer extension products, produced from a template vector, said products containing sequences corresponding or complementary to i) a primer binding region, ii) an insert and iii) a vector-derived sequence(s) which is derived from any portion of said template vector excluding said primer binding region or said insert, wherein said method comprises at least the step or steps of binding a modular oligonucleotide composed of at least two separate oligonucleotides (modules) to adjacent stretches on said primer extension products, wherein said modular oligonucleotide composed of said modules is complementary to and capable of binding to said vector-derived sequence of said primer extension products, wherein at least one module (capture module) which is immobilized or has means for immobilization, and further wherein said vector-derived sequence(s) of said primer extension products is/are derived from any region of the vector used to produce the template vector and contains a portion which remains intact after cloning of the insert, regardless of restriction site of a multiple cloning site of said vector into which the insert is cloned, and to which the modular oligonucleotide anneals, and wherein binding of the modular oligonucleotide and the primer binding site and/or insert is not sufficient to allow capture of primers and/ or misprimed products, and further wherein the method results in captured primer extension products.

2. A method as claimed in claim 1, wherein said vector-derived sequence(s) of said primer extension products is derived from any region of the vector used to produce the template vector and contains a portion which remains intact after cloning of the insert into a particular restriction site of the multiple cloning site of said vector and to which the modular oligonucleotide anneals.

3. A method as claimed in claim 1 or claim 2 wherein said modular oligonucleotide consists of two or three modules.

4. A method as claimed in any of claims 1 to 3 wherein each module has ≥9 ≤ 18 nucleotides.

5. A method as claimed in any of claims 1 to 4 wherein said modular oligonucleotide consists of a total of at least 18 nucleotides.

6. A method as claimed in any of claims 1 to 5 wherein the modules when bound to said primer extension products are less than 2 bases apart.

7. A method as claimed in claim 6 wherein said modules are directly adjacent.

8. A method as claimed in any of claims 1 to 7 wherein immobilization is via the streptavidin:biotin binding system.

9. A method as claimed in any of claims 1 to 8 wherein said primer extension products are contacted directly with all modules of said modular oligonucleotide in a single hybridization step.

10. A method as claimed in any of claims 1 to 9 comprising the steps of:
1) contacting the sample containing the primer extension products with all modules of the modular oligonucleotide, wherein the capture module is immobilized on a solid support;
2) binding said modules by hybridization;
3) separating target primer extension products bound to said solid support; and
4) washing said solid support.

11. A method as claimed in any of claims 1 to 10 wherein said binding is performed at between 40°C. and 60°C. for between 15 to 90 minutes.

12. A method as claimed in any of claims 1 to 11 wherein said modular oligonucleotide is selected from:
1) 5'-NNAAGCTTACT-3' (capture module) and 5'-GGCCGTCGTTTTACAACG-3'
2) 5'-NNAAGCTTGGCACT-3' (capture module) and 5'-GGCCGTCGTTTTACAACG-3'
3) 5'-NNNNAATTCGCCCTATAG-3' (capture module) and 5'-TGAGTCGTATTAC-3'
4) 5'-GAATTCACGGAAATCATG-3' (capture module) and 5'-GTCATAGCTGTTTCCTGT-3'
5) 5'-NNGAATTCGTAATCATGGTC-3' (capture module) and 5'-ATAGCTGTTTCCTGTGT-3'
6) 5'-TCCAGCTTTTGTTCC-3' (capture module) and 5'-CTTTAGTGAGGGTTAATT-3'
7) 5'-AGCTCCAGCTTTTGTTCC-3' (capture module) and 5'-CTTTAGTGAGGGTTAATT-3'
8) 5'-NNTTGAGTATTCTATAG-3' (capture module) and 5'-TGTCACCTAAATAGCT-3'
9) 5'-TCTAGAGTCGACCTGCAG-3' (capture module) and 5'-GCATGCAAGCTT-3'
10) 5'-ACCCAATTCGCCCTATAG-3' (capture module) and 5'-TGAGTCGTATTAC-3' and
11) 5'-CGAGCTCGAATTCGTAATC-3' (capture module) and 5'-ATGGTCATAGCTGTTTCC-3',
or analogs or derivatives thereof with modified or derivatized nucleotide bases but which retain their complementarity.

13. A method as claimed in any one of claims 1 to 12, wherein said primer extension products are sequencing reaction products.

14. A modular oligonucleotide selected from:
1) 5'-NNAAGCTTACT-3' (capture module) and 5'-GGCCGTCGTTTTACAACG-3'
2) 5'-NNAAGCTTGGCACT-3' (capture module) and 5'-GGCCGTCGTTTTACAACG-3'
3) 5'-NNNNAATTCGCCCTATAG-3' (capture module) and 5'-TGAGTCGTATTAC-3'
4) 5'-GAATTCACGGAAATCATG-3' (capture module) and 5'-GTCATAGCTGTTTCCTGT-3'
5) 5'-NNGAATTCGTAATCATGGTC-3' (capture module) and 5'-ATAGCTGTTTCCTGTGT-3'
6) 5'-TCCAGCTTTTGTTCC-3' (capture module) and 5'-CTTTAGTGAGGGTTAATT-3'
7) 5'-AGCTCCAGCTTTTGTTCC-3' (capture module) and 5'-CTTTAGTGAGGGTTAATT-3'
8) 5'-NNTTGAGTATTCTATAG-3' (capture module) and 5'-TGTCACCTAAATAGCT-3'

15. A kit for performing the method as defined in any one of claims 1 to 13, comprising at least the following:
a modular oligonucleotide as defined in claim 14.

16. A method of determining the nucleotide sequence of a nucleic acid insert in a vector wherein sequencing products are generated by performing appropriate extension reactions on said vector, the sequencing products are captured by a method as claimed in any of claims 1 to 13, the products thus captured are separated by an appropriate technique and the labels carried on said sequencing products are visualized to allow determination of the sequence of said insert or a portion thereof.

17. A method as claimed in any one of claims 1 to 13 or 16, wherein said binding step is performed in the presence of polyethylene glycol.

## Patentansprüche

1. Ein Verfahren zur Erfassung von Primer-Verlängerungsprodukten, die aus einem Template-Vektor hergestellt wurden, wobei die Produkte Sequenzen enthalten, die i) einer Primer-Bindungsregion, ii) einer Insertion und iii) einer von einem Vektor abgeleiteten Sequenz (Sequenzen), die sich von einem beliebigen Teil des Template-Vektors mit Ausnahme der Primer-Bindungsregion oder der Insertion ableitet, entsprechen oder dazu komplementär sind, wobei das Verfahren mindestens den Schritt oder die Schritte der Bindung eines modularen Oligonukleotids, das aus mindestens zwei separaten Oligonukleotiden (Modulen) aufgebaut ist, an benachbarte Abschnitte auf den Primer-Verlängerungsprodukten umfasst, wobei das modulare Oligonukleotid, das aus den Modulen aufgebaut ist, zu der von einem Vektor abgeleiteten Sequenz der Primer-Verlängerungsprodukte komplementär und in der Lage ist, an diese zu binden, wobei mindestens ein Modul (Erfassungsmodul), das immobilisiert ist oder Mittel zur Immobilisierung aufweist, und wobei ferner die von einem Vektor abgeleitete Sequenz (abgeleiteten Sequenzen) der Primer-Verlängerungsprodukte von einer beliebigen Region des Vektors abgeleitet ist/sind, der zur Herstellung des Template-Vektors verwendet wurde und einen Abschnitt enthält, der nach Klonen der Insertion intakt bleibt, ungeachtet der Restriktionsstelle einer mehrfachen Klonierungsstelle des Vektors, in den die Insertion kloniert wird, und an den das modulare Oligonukleotid anlagert, und wobei die Bindung des modularen Oligonukleotids und die Primer-Bindungsstelle und/oder Insertion nicht ausreicht, um eine Erfassung von Primern und/oder misprimed Produkten zu gestatten, und wobei ferner das Verfahren zu erfassten Primer-Verlängerungsprodukten führt.

2. Ein Verfahren nach Anspruch 1, wobei die von einem Vektor abgeleitete Sequenz (abgeleiteten Sequenzen) der Primer-Verlängerungsprodukte von einer beliebigen Region des Vektors abgeleitet ist/sind, der zur Herstellung des Template-Vektors verwendet wurde, und einen Abschnitt enthält, der nach Klonen der Insertion in eine bestimmte Restriktionsstelle der mehrfachen Klonierungsstelle des Vektors intakt bleibt und an den das modulare Oligonukleotid anlagert.

3. Ein Verfahren nach Anspruch 1 oder 2, wobei das modulare Oligonukleotid aus zwei oder drei Modulen besteht.

4. Ein Verfahren nach einem der Ansprüche 1 bis 3, wobei jedes Modul ≥ 9 ≤ 18 Nukleotide aufweist.

5. Ein Verfahren nach einem der Ansprüche 1 bis 4, wobei das modulare Oligonukleotid aus insgesamt mindestens 18 Nukleotiden besteht.

6. Ein Verfahren nach einem der Ansprüche 1 bis 5, wobei die Module weniger als 2 Basen entfernt sind, wenn sie an die Primer-Verlängerungsprodukte gebunden sind.

7. Ein Verfahren nach Anspruch 6, wobei die Module direkt benachbart sind.

8. Ein Verfahren nach einem der Ansprüche 1 bis 7, wobei die Immobilisierung über das Streptavidin:Biotin-Bindungssystem erfolgt.

9. Ein Verfahren nach einem der Ansprüche 1 bis 8, wobei die Primer-Verlängerungsprodukte direkt mit sämtlichen Modulen des modularen Oligonukleotids in einem einzigen Hybridisierungsschritt in Kontakt gebracht werden.

10. Ein Verfahren nach einem der Ansprüche 1 bis 9, das die folgenden Schritte umfasst:
1) Inkontaktbringen der Probe, die die Primer-Verlängerungsprodukte enthält, mit sämtlichen Modulen des modularen Oligonukleotids, wobei das Erfassungsmodul auf einem festen Träger immobilisiert ist;
2) Binden der Moleküle durch Hybridisierung;
3) Abbrennen der Ziel-Primer-Verlängerungsprodukte, die an dem festen Träger gebunden sind; und
4) Waschen des festen Trägers.

11. Ein Verfahren nach einem der Ansprüche 1 bis 10, wobei die Bindung zwischen 40 °C und 60 °C zwischen 15 bis 90 Minuten durchgeführt wird.

12. Ein Verfahren nach einem der Ansprüche 1 bis 11, wobei das modulare Oligonukleotid aus folgendem ausgewählt ist:
1) 5'-NNAAGCTTACT-3' (Erfassungs modul) und 5'-GGCCGTCGTTTTACAACG-3'
2) 5'-NNAAGCTTGGCACT-3' (Erfassungsmodul) und 5'-GGCCGTCGTTTTACAACG-3'
3) 5'-NNNNAATTCGCCCTATAG-3' (Erfassungsmodul) und 5'-TGAGTCGTATTAC-3'
4) 5'-GAATTCACGGAAATCATG-3' (Erfassungsmodul) und 5'-GTCATAGCTGTTTCCTGT-3'
5) 5'-NNGAATTCGTAATCATGGTC-3' (Erfassungsmodul) und 5'-ATAGCTGTTTCCTGTGT-3'
6) 5'-TCCAGCTTTTGTTCC-3' (Erfassungsmodul) und 5'-CTTTAGTGAGGGTTAATT-3'
7) 5'-AGCTCCAGCTTTTGTTCC-3' (Erfassungsmodul) und 5'-CTTTAGTGAGGGTTAATT-3'
8) 5'-NNTTGAGTATTCTATAG-3' (Erfassungsmodul) und 5'-TGTCACCTAAATAGCT-3'
9) 5'-TCTAGAGTCGACCTGCAG-3' (Erfassungsmodul) und 5'-GCATGCAAGCTT-3'
10) 5'-ACCCAATTCGCCCTATAG-3' (Erfassungsmodul) und 5'-TGAGTCGTATTAC-3' und
11) 5'-CGAGCTCGAATTCGTAATC-3' (Erfassungsmodul) und 5'-ATGGTCATAGCTGTTTCC-3',
oder Analoga oder Derivaten davon mit modifizierten oder derivatisierten Nukleotidbasen, die jedoch ihre Komplementarität behalten.

13. Ein Verfahren nach einem der Ansprüche 1 bis 12, wobei die Primer-Verlängerungsprodukte Sequenzierungsreaktionsprodukte sind.

14. Ein modulares Oligonukleotid, das aus folgendem ausgewählt ist:
1) 5'-NNAAGCTTACT-3' (Erfassungsmodul) und 5'-GGCCGTCGTTTTACAACG-3'
2) 5'-NNAAGCTTGGCACT-3' (Erfassungsmodul) und 5'-GGCCGTCGTTTTACAACG-3'
3) 5'-NNNNAATTCGCCCTATAG-3' (Erfassungsmodul) und 5'-TGAGTCGTATTAC-3'
4) 5'-GAATTCACGGAAATCATG-3' (Erfassungsmodul) und 5'-GTCATAGCTGTTTCCTGT-3'
5) 5'-NNGAATTCGTAATCATGGTC-3' (Erfassungsmodul) und 5'-ATAGCTGTTTCCTGTGT-3'
6) 5'-TCCAGCTTTTGTTCC-3' (Erfassungsmodul) und 5'-CTTTAGTGAGGGTTAATT-3'
7) 5'-AGCTCCAGCTTTTGTTCC-3' (Erfassungsmodul) und 5'-CTTTAGTGAGGGTTAATT-3'
8) 5'-NNTTGAGTATTCTATAG-3' (Erfassungsmodul) und 5'-TGTCACCTAAATAGCT-3'.

15. Ein Kit zur Durchführung des Verfahrens, wie in einem der Ansprüche 1 bis 13 definiert, das mindestens folgendes umfasst:
ein modulares Oligonukleotid, wie in Anspruch 14 definiert.

16. Ein Verfahren zur Bestimmung der Nukleotidsequenz einer Nukleinsäure-Insertion in einem Vektor, wobei die Sequenzierungsprodukte über die Durchführung zweckmäßiger Verlängerungsreaktionen an dem Vektor erzeugt werden, die Sequenzierungsprodukte durch ein Verfahren nach einem der Ansprüche 1 bis 13 erfasst werden, die so erfassten Produkte durch ein zweckmäßiges Verfahren getrennt werden und die Markierungen, die an den Sequenzierungsprodukten getragen werden, sichtbar gemacht werden, um eine Bestimmung der Sequenz der Insertion oder eines Abschnitts davon zu gestatten.

17. Ein Verfahren nach einem der Ansprüche 1 bis 13 oder 16, wobei der Bindungsschritt in Gegenwart von Polyethylenglycol durchgeführt wird.

## Revendications

1. Procédé pour capturer des produits d'extension d'amorce, fabriqués à partir d'un vecteur de modèle, lesdits produits contenant des séquences correspondant ou complémentaires à : i) une région de liaison d'amorce, ii) un insert et iii) une ou des séquences issues d'un vecteur qui sont issues de n'importe quelle partie dudit vecteur de modèle excluant ladite région de liaison d'amorce ou ledit insert, ledit procédé comprenant au moins la étape ou les étapes consistant à lier un oligonucléotide modulaire composé d'au moins deux (modules) oligonucléotides séparés à des étirements adjacents sur lesdits produits d'extension d'amorce, ledit oligonucléotide modulaire composé desdits modules étant complémentaire à et apte à se lier à ladite séquence issue d'un vecteur desdits produits d'extension d'amorce, au moins un module (module de capture) étant immobilisé ou ayant des moyens d'immobilisation, et, en outre, ladite ou lesdites séquences issues d'un vecteur desdits produits d'extension d'amorce étant issues de n'importe quelle région du vecteur utilisé pour fabriquer le vecteur de modèle et contenant une partie qui reste intacte après le clonage de l'insert, indépendamment du site de restriction d'un site de clonage multiple dudit vecteur dans lequel l'insert est cloné, et à laquelle l'oligonucléotide modulaire s'annèle, et la liaison de l'oligonucléotide modulaire et du site de liaison d'amorce et/ou de l'insert n'étant pas suffisante pour permettre de capturer des amorces et/ou des produits avec la mauvaise amorce, et le procédé conduisant en outre à des produits d'extension d'amorce capturés.

2. Procédé selon la revendication 1, dans lequel ladite ou lesdites séquences issues d'un vecteur desdits produits d'extension d'amorce sont issues de n'importe quelle région du vecteur utilisé pour fabriquer le vecteur de modèle et contiennent une partie qui reste intacte après le clonage de l'insert dans un site de restriction particulier du site de clonage multiple dudit vecteur et à laquelle l'oligonucléotide s'annèle.

3. Procédé selon l'une des revendications 1 ou 2, dans lequel ledit oligonucléotide modulaire consiste en deux ou trois modules.

4. Procédé selon l'une quelconque des revendications 1 à 3, dans lequel chaque module a ≥9 ≤18 nucléotides.

5. Procédé selon l'une quelconque des revendications 1 à 4, dans lequel ledit oligonucléotide modulaire consiste en un total d'au moins 18 nucléotides.

6. Procédé selon l'une quelconque des revendications 1 à 5, dans lequel les modules, lorsqu'ils sont liés auxdits produits d'extension d'amorce, sont séparés de moins de 2 bases.

7. Procédé selon la revendication 6, dans lequel lesdits modules sont directement adjacents.

8. Procédé selon l'une quelconque des revendications 1 à 7, dans lequel l'immobilisation se fait par l'intermédiaire du système de liaison streptavidine : biotine.

9. Procédé selon l'une quelconque des revendications 1 à 8, dans lequel lesdits produits d'extension d'amorce sont directement mis en contact avec tous les modules dudit oligonucléotide modulaire en une seule étape d'hybridation.

10. Procédé selon l'une quelconque des revendications 1 à 9, comprenant les étapes consistant à :
1) mettre en contact l'échantillon contenant les produits d'extension d'amorce avec tous les modules de l'oligonucléotide modulaire, le module de capture étant immobilisé sur un support solide ;
2) lier lesdits modules par hybridation ;
3) séparer les produits d'extension d'amorce cibles liés audit support solide ; et
4) laver ledit support solide.

11. Procédé selon l'une quelconque des revendications 1 à 10, dans lequel ladite liaison est réalisée entre 40°C et 60°C pendant 15 à 90 minutes.

12. Procédé selon l'une quelconque des revendications 1 à 11, dans lequel ledit oligonucléotide modulaire est choisi parmi :
1) 5'-NNAAGCTTACT-3' (module de capture) et 5'-GGCCGTCGTTTTACAACG-3'
2) 5'-NNAAGCTTGGCACT-3' (module de capture) et 5'-GGCCGTCGTTTTACAACG-3'
3) 5'-NNNNAATTCGCCCTATAG-3' (module de capture) et 5'-TGAGTCGTATTAC-3'
4) 5'-GAATTCACGGAAATCATG-3' (module de capture) et 5'-GTCATAGCTGTTTCCTGT-3'
5) 5'-NNGAATTCGTAATCATGGTC-3' (module de capture) et 5'-ATAGCTGTTTCCTGTGT-3'
6) 5'-TCCAGCTTTTGTTCC-3' (module de capture) et 5'-CTTTAGTGAGGGTTAATT-3'
7) 5'-AGCTCCAGCTTTTGTTCC-3' (module de capture) et 5'-CTTTAGTGAGGGTTAATT-3'
8) 5'-NNTTGAGTATTCTATAG-3' (module de capture) et 5'-TGTCACCTAAATAGCT-3'
9) 5'-TCTAGAGTCGACCTGCAG-3' (module de capture) et 5'-GCATGCAAGCTT-3'
10) 5'-ACCCAATTCGCCCTATAG-3' (module de capture) et 5'-TGAGTCGTATTAC-3' et
11) 5'-CGAGCTCGAATTCGTAATC-3' (module de capture) et 5'-ATGGTCATAGCTGTTTCC-3',
ou des analogues ou dérivés de ceux-ci avec des bases de nucléotides modifiés ou dérivés mais qui conservent leur complémentarité.

13. Procédé selon l'une quelconque des revendications 1 à 12, dans lequel lesdits produits d'extension d'amorce sont des produits de réaction de séquençage.

14. Oligonucléotide choisi parmi :
1) 5'-NNAAGCTTACT-3' (module de capture) et 5'-GGCCGTCGTTTTACAACG-3'
2) 5'-NNAAGCTTGGCACT-3' (module de capture) et 5'-GGCCGTCGTTTTACAACG-3'
3) 5'-NNNNAATTCGCCCTATAG-3' (module de capture) et 5'-TGAGTCGTATTAC-3'
4) 5'-GAATTCACGGAAATCATG-3' (module de capture) et 5'-GTCATAGCTGTTTCCTGT-3'
5) 5'-NNGAATTCGTAATCATGGTC-3' (module de capture) et 5'-ATAGCTGTTTCCTGTGT-3'
6) 5'-TCCAGCTTTTGTTCC-3' (module de capture) et 5'-CTTTAGTGAGGGTTAATT-3'
7) 5'-AGCTCCAGCTTTTGTTCC-3' (module de capture) et 5'-CTTTAGTGAGGGTTAATT-3'
8) 5'-NNTTGAGTATTCTATAG-3' (module de capture) et 5'-TGTCACCTAAATAGCT-3'

15. Coffret pour effectuer le procédé tel que défini selon l'une quelconque des revendications 1 à 13, comprenant au moins un oligonucléotide modulaire tel que défini à la revendication 14.

16. Procédé de détermination de la séquence nucléotidique d'un insert d'acide nucléique dans un vecteur, dans lequel des produits de séquençage sont générés par la réalisation de réactions d'extension appropriées sur ledit vecteur, les produits de séquençage sont capturés par un procédé selon l'une quelconque des revendications 1 à 13, les produits ainsi capturés sont séparés par une technique appropriée et les marqueurs portés sur lesdits produits de séquençage sont visualisés pour permettre la détermination de la séquence dudit insert ou d'une partie de celui-ci.

17. Procédé selon l'une quelconque des revendications 1 à 13 ou 16, dans lequel ladite étape de liaison est effectuée en présence de polyéthylène glycol.
